# EUROPEAN PATENT SPECIFICATION

(11) **EP 2 890 451 B1**
(45) Date of publication and mention of the grant of the patent: **22.09.2021**
(21) Application number: 13760181.1
(22) Date of filing: 28.08.2013
(51) Int. Cl.: A61N 1/36, A61N 1/05, A61N 1/372

(54) **PARAMETER VISUALIZATION, SELECTION, AND ANNOTATION INTERFACE**
SCHNITTSTELLE FÜR PARAMETERVISUALISIERUNG, AUSWAHL UND KOMMENTIERUNG
VISUALISATION DE PARAMÈTRES, INTERFACE DE SÉLECTION ET D'ANNOTATION

(30) Priority: 28.08.2012 US 201261693866 P; 10.09.2012 US 201261699135 P; 10.09.2012 US 201261699115 P; 16.01.2013 US 201361753232 P
(43) Date of publication of application: 08.07.2015
(73) Proprietor: Boston Scientific Neuromodulation Corporation, Valencia, CA 91355 (US)
(72) Inventor: CARCIERI, Stephen, Los Angeles, California 90026 (US); CHEN, Dean, Irvine, California 92603 (US); MOFFITT, Michael A., Valencia, California 91354 (US)
(74) Representative: Vossius & Partner Patentanwälte Rechtsanwälte mbB
(86) International application number: PCT/US2013/056981
(87) International publication number: WO 2014/036079

(56) References cited:
- WO-A1-2007/097858
- WO-A1-2012/057951
- US-A1- 2009 287 271
- None

## Description

### FIELD OF THE INVENTION

Example embodiments of the present invention relate to a system and method for providing a user interface in which a representation of stimulation parameters of an electrode leadwire, for example that provides electrical stimulation to an anatomical region, e.g., a leadwire-of a Deep Brain Stimulation (DBS) device or a Spinal Cord Stimulation (SCS) device, is provided. Example embodiments of the present invention further relate additionally or alternatively to providing the user interface via which to set and annotate such parameters. Example embodiments of the present invention further relate additionally or alternatively to a system and method using software which provides a visual point-and-click interface that allows a user to optimize a subject's (e.g., patient's) stimulation parameters, without the user having to keep track of the precise settings for each electrode. Example embodiments of the present invention further relate additionally or alternatively to a system and method for generating and outputting clinical effects and/or side-effects maps as a visual history of anatomic tissue stimulations provided by an electrode leadwire, e.g., of a Deep Brain Stimulation (DBS) device or a Spinal Cord Stimulation (SCS) device. Such maps can be provided on a patient-specific basis. Example embodiments of the present invention further relate additionally or alternatively to methods and systems for determining target stimulation electrical stimulation parameters of an anatomical region of the body.

### BACKGROUND

Stimulation of anatomical regions of a patient is a clinical technique for the treatment of disorders. Such stimulation can include deep brain stimulation (DBS), spinal cord stimulation (SCS), Occipital NS therapy, Trigemenal NS therapy, Vagus NS therapy, peripheral field stimulation therapy, sacral root stimulation therapy, or other such therapies. For example, DBS may include stimulation of the thalamus or basal ganglia and may be used to treat disorders such as essential tremor, Parkinson's disease (PD), and other physiological disorders, including psychiatric disorders. DBS may also be useful for traumatic brain injury and stroke. Pilot studies have also begun to examine the utility of DBS for treating dystonia, epilepsy, and obsessive-compulsive disorder.

However, understanding of the therapeutic mechanisms of action remains elusive. The stimulation parameters, electrode geometries, or electrode locations that are best suited for existing or future uses of DBS also are unclear.

For conducting a therapeutic stimulation, a neurosurgeon can select a target region within the patient anatomy, e.g., within the brain for DBS, an entry point, e.g., on the patient's skull, and a desired trajectory between the entry point and the target region. The entry point and trajectory are typically carefully selected to avoid intersecting or otherwise damaging certain nearby critical structures or vasculature. A stimulation electrode leadwire used to provide the stimulation to the relevant anatomical region is inserted along the trajectory from the entry point toward the target region. The stimulation electrode leadwire typically includes multiple closely-spaced electrically independent stimulation electrode contacts.

The target anatomical region can include tissue that exhibit high electrical conductivity. For a given stimulation parameter setting, a respective subset of the fibers are responsively activated. A stimulation parameter can include a current amplitude or voltage amplitude, which may be the same for all of the electrodes of the leadwire, or which may vary between different electrodes of the leadwire. The applied amplitude setting results in a corresponding current in the surrounding fibers, and therefore a corresponding voltage distribution in the surrounding tissue. The complexity of the inhomogeneous and anisotropic fibers makes it difficult to predict the particular volume of tissue influenced by the applied stimulation.

A treating physician typically would like to tailor the stimulation parameters (such as which one or more of the stimulating electrode contacts to use, the stimulation pulse amplitude, e.g., current or voltage depending on the stimulator being used, the stimulation pulse width, and/or the stimulation frequency) for a particular patient to improve the effectiveness of the therapy. Parameter selections for the stimulation can be achieved via tedious and variable trial-and-error, without visual aids of the electrode location in the tissue medium or computational models of the volume of tissue influenced by the stimulation. Such a method of parameter selection is difficult and time-consuming and, therefore, expensive. Moreover, it may not necessarily result in the best possible therapy.

Systems have been proposed that provide an interface that facilitates parameter selections. See, for example, U.S. Pat. App. Ser. No. 12/454,330, filed May 15, 2009 ("the '330 application"), U.S. Pat. App. Ser. No. 12/454,312, filed May 15, 2009 ("the '312 application"), U.S. Pat. App. Ser. No. 12/454,340, filed May 15, 2009 ("the '340 application"), U.S. Pat. App. Ser. No. 12/454,343, filed May 15, 2009 ("the '343 application"), and U.S. Pat. App. Ser. No. 12/454,314, filed May 15, 2009 ("the '314 application").

The leadwire can include cylindrically symmetrical electrodes, which, when operational, produce approximately the same electric values in all positions at a same or-similar distance from the electrode in any plane that cuts through the electrode. Alternatively, the leadwire can include directional electrodes that produce different electrical values depending on the direction from the electrode. For example, the leadwire can include multiple separately controllable electrodes arranged cylindrically about the leadwire at each of a plurality of levels of the leadwire. Each electrode may be set as an anode or cathode in a bipolar configuration or as a cathode, with, for example, the stimulator casing being used as ground, in a monopolar arrangement.

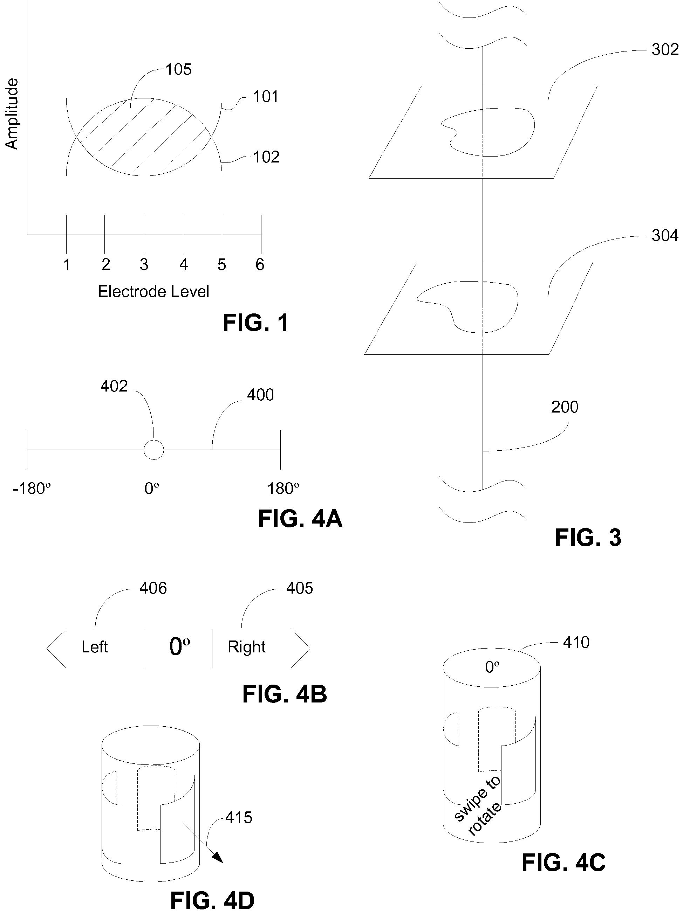

When programming a leadwire for tissue stimulation, e.g., DBS, the clinical standard of care is often to perform a monopolar review (MPR) upon activation of the leadwire in order to determine the efficacy and side-effect thresholds for all electrodes on the leadwire, on an electrode-by-electrode basis. Monopolar review, rather than bipolar review, is performed because monopolar stimulation often requires a lower stimulation intensity than bipolar stimulation to achieve the same clinical benefit. The MPR can inform the selection of a first clinical program (parameters for stimulation) for treating a patient. For example, in a single current source, voltage-controlled DBS device, a time-consuming review is performed involving sequentially measuring efficacy and side-effect thresholds for all electrodes of a leadwire and recording these threshold values. Such a tedious review is described in Volkmann et al., Introduction to the Programming of Deep Brain Stimulators, Movement Disorders Vol. 17, Suppl. 3, pp. S181-S187 (2002) ("Volkmann"). See, for example, figure 3 of Volkmann and the corresponding text, which describes gradually increasing amplitude separately for each of a plurality of electrodes, and recording the amplitude at which a minimum threshold of therapeutic efficacy is observed, and the maximum amplitude that does not exceed a permitted adverse side-effect threshold.

Such systems for programming a leadwire for tissue stimulation display a graphical representation of an area within which it is estimated that there is or could be tissue activation, referred to herein as a volume of activation (VOA), that results from input stimulation parameters. For example, the VOA can be calculated as a region outside of which stimulation is estimated to be unlikely. The VOA can be displayed relative to an image or model of a portion of the patient's anatomy.

Generation of the VOA may be based on Neural Element Models such as a model of fibers, e.g., axons, and a voltage distribution about the leadwire and on detailed processing thereof. Performing such processing to provide a VOA preview in real-time response to a clinician's input of parameters is not practical because of the significant required processing time. Therefore, conventional systems pre-process various stimulation parameter settings to determine which axons are activated by the respective settings.

Those systems also provide interfaces via which to input selections of the stimulation parameters and notes concerning therapeutic and/or side effects of stimulations associated with graphically represented VOAs. Document US-A-2009/287271 discloses the most relevant prior art.

### SUMMARY

The invention is defined in claim 1. Any embodiment which is in contradiction to the subject-matter of claim 1 is not part of the invention.

According to example embodiments of the present invention, the VOA is a two-dimensional or three-dimensional model. For example, in an example embodiment, the VOA is a three-dimensional model composed of voxels, a volume element within a three-dimensional grid. The VOA identifies a tissue region that is estimated to be active for a given stimulation.

In an example embodiment, the system records in a memory clinical effects of a stimulations represented by a respective VOA are recorded. For example, one or more sensors are used to obtain physiological information of a patient on whom a stimulation is performed, which stimulation is estimated by the system to produce a VOA, with which VOA the sensor information is used. Alternatively or additionally, the system includes a user interface via which the system is configured to receive user input of clinical effects information for a given stimulation which the system stores in association with a VOA which the system estimates for the stimulation.

In an example embodiment, the system assigns a score to the VOA based on such clinical effects data. In an example embodiment, the system assigns individual pixels (or pixel regions) and/or three-dimensional voxels (or voxel regions) a score based on clinical effects data of the VOA(s) of which they have been a part, e.g., based on scores of the VOA(s) of which they have been a part. For example, in an example embodiment, the pixel and/or voxel scores are based on any combination of the clinical data for the graphical regions, for example, an average of recorded values.

According to an alternative example embodiment, Neural Element Models are directly scored in a manner to that described above for voxels. These models are expected to be more faithful to neural function.

According to example embodiments of the present invention, a leadwire includes multiple electrodes, for each of which a respective independent current source is provided, by which current can be "steered" longitudinally and/or rotationally about the leadwire for localization of stimulation at points along the lead wire. Example embodiments of the present invention provide a visual interface that includes a graphical representation of-the effects of stimulation on the anatomy of a particular patient or group of patients. However, prior to initial programming of the leadwire, no direct information about a patient's response to stimulation is known. In an example embodiment, the absence of information is also represented. For example, in an example embodiment, the system displays an anatomical map with a "fog-of-war" feature by which areas of the anatomical map for which a VOA has been estimated for a conducted stimulation are displayed in a fully revealed manner, whereas other areas of the anatomical map are left blacked out or in a haze, e.g., with less brightness. In an example embodiment, the system displays in a fully revealed manner only those regions corresponding to VOAs associated with stimulations for which the system has obtained clinical effects information, e.g., via sensor or via user input. Such a map can be generated for a patient population. In an example embodiment, the map is patient specific. (While the described embodiments refer to an anatomical map, e.g., formed of an anatomical atlas background or a background of medical images, in alternative example embodiments, the map is of a region relative to an implanted leadwire, e.g., even without representations of anatomical structures, and it is to be understood that the described features can be implemented with such a leadwire centric map.)

For example, in an example embodiment, the system generates an anatomical map with graphical indicia for distinguishing between different regions of the map based on clinical effect, where the respective clinical effect indicated for a respective region depends on the clinical effects information obtained by the system for a VOA estimated to include the respective region, and further based on whether the respective region has been included in such a VOA. Such indicia can include variations in color, brightness, hatching, transparency, shading, etc. According to an example embodiment, the map includes areas that are not revealed to the user until information regarding the effects of stimulation in that anatomical area have been obtained by exploring the area with electrical stimulation. For example, the region is blacked out. Subsequent to performance of a stimulation whose estimated VOA covers the region, and, for example, for which VOA clinical effects information is obtained, the formerly blacked out region which is part of the VOA is displayed in a non-blacked out manner in a subsequently generated information map. In an example embodiment, the region is displayed in the non-blacked out manner, even if clinical effects information is not obtained. However, graphical indicia representing respective clinical effects information is not displayed for the now non-blacked out region until such information is obtained for a VOA inclusive of the respective region.

The clinical effects information can include therapeutic effect information indicating the effectiveness of respective stimulations associated with VOAs for providing a therapeutic effect and/or adverse side-effect information indicating adverse side effects caused by respective stimulations associated with VOAs. The clinical effects information is captured and made available to the user in the map to provide a visualization of the degree of therapeutic effect and/or adverse side effect using gradations in graphical indicia within the map. Such a map can help facilitate the subsequent choosing of stimulation parameters for programming the leadwire in a more informed way. For example, parameters can be selected which are estimated to produce a VOA corresponding to a target region, which target region is selected as a portion of the map graphically associated with a certain level of therapeutic effect and with less than some maximum tolerated adverse side effect.

According to example embodiments of the present invention, the graphical representation of the effects of stimulation on the anatomy of a particular group of patients (or a specific patient) includes a mean (or other statistical combination) of clinical and/or side-effect values obtained for each anatomical location. These mathematical methods may be less useful for single-patient representations because of the small data sampling on which the base the map, but it is still possible. According to example embodiments of the present invention, the graphical representation of the effects of stimulation on the anatomy of a patient population of a particular patient includes a maximum or minimum value of therapeutic effect and/or side-effect values obtained for each anatomical location. For example, a voxel can be part of a plurality of different VOAs associated with different sets of stimulation parameters that have been used to perform respective stimulations for which different clinical effects information had been obtained, and the map, according to an example embodiment, provides graphical indicia at the voxel based on the VOA for which a maximum score, or, alternatively, a minimum score had been obtained for a therapeutic effect, or, alternatively, an adverse side effect. Such information can be based on all therapeutic or adverse side effects or for a selected one or more particular therapeutic and/or side effects.

Use of maximum or minimum score values for a voxel provides an advantage of informing the user of threshold values that are required to achieve a therapeutic effect or to avoid an adverse side-effect. However, this can also have the effect of diluting the importance of a location that may have appeared more promising using a more complex statistical analysis which takes into account a combination of scores with which the voxel is associated.

According to example embodiments of the present invention, the graphical representation of the effects of stimulation on the anatomy of a particular patient includes an overall value (therapeutic effect + side-effect) of therapeutic and/or side-effect values obtained for each anatomical location based on historical stimulations of the patient, in order to accomplish the dual objective of maximizing clinical effect and minimizing adverse side-effects. For example, the system calculates a blended score. According to alternative example embodiments of the present invention, the graphical representation of the effects of stimulation on the anatomy of a particular group of patients includes a maximum or minimum value of an overall value (therapeutic effect + side-effect) of therapeutic and/or side-effect values obtained for each anatomical location based on historical stimulations of a patient population, e.g., of all recorded patient stimulations or of those of a selected sub-group of the patient population, e.g., selected based on medical indications and/or patient demographics.

Thus, according to example embodiments of the present invention, the graphical representation of the effects of stimulation on the anatomy of a particular patient includes an estimated therapeutic effect and/or side-effect value for a given location based on known information, e.g. statistical data for the anatomical location compared to a library of data.

According to example embodiments of the present invention, the graphical representation of the effects of stimulation on the anatomy of a particular patient includes a graphical representation of a binary (OK, Not OK) side-effects threshold for a given location based on known information. According to example embodiments of the present invention, the graphical representation of a binary (OK, Not OK) side-effects threshold for a given location is overlaid on other graphical indicia of therapeutic effects of stimulation on the anatomy of a particular patient or group of patients. According to alternative example embodiments of the present invention, the representations of the side-effects values that are overlaid on the graphical indicia of the therapeutic effects indicate a plurality of side effect scores, (e.g., using different hatching or dot densities).

According to example embodiments of the present invention, the graphical representation of the effects of stimulation on the patient anatomy includes three-dimensional representations of clinical effects volumes, for example, in which the clinical effect values are represented as three-dimensional surfaces based on threshold values for therapeutic effects and/or adverse side-effects. These 3-D surfaces can include some transparency so that anatomical and/or leadwire features are not completely obscured. The "fog-of-war" regions (the regions not explored with stimulations) in three dimensions is represented as, for example, a darkness or fog which can include some transparency so that anatomical and/or leadwire features are not completely obscured.

According to an alternative example embodiment, the graphical representation of the effects of stimulation on the anatomy includes two-dimensional representations of clinical effects volumes in which the clinical effect values are represented as 2-D planes indicated with, e.g., color, hatching, patterning, etc., and the user can select which plane is being displayed, for example, by selection of plane direction, and plane slice in the selected direction. The "fog-of-war" in 2 dimensions is represented as, for example, a color (e.g., black or white) or patterning.

According to example embodiments of the present invention, the graphical representation of the effects of stimulation on the anatomy includes three-dimensional representations of a volume including therein modeled anatomical fibers, e.g., modeled neurons of a Neural Element Model (NEM), presented as a 3-D map in which the clinical effect values are represented as lines or 3-D neural objects graphically distinguished from each other based on estimated activation regions for respective stimulations for which clinical effects information had been previously obtained. These 3-D objects can include some transparency so that anatomical and/or leadwire features are not completely obscured. The "fog-of-war" in 3 dimensions is represented as, for example, a darkness or fog which can include some transparency so that anatomical features, including modeled anatomical fibers, and/or leadwire features are not completely obscured. According to example embodiments of the present invention, the graphical representation of the effects of stimulation includes two-dimensional representations of clinical effects volumes graphically representing the anatomical fibers, where the clinical effect values are represented with, e.g., color, hatching, patterning, etc., of the two-dimensionally rendered fiber models, where the user can select the plane to be displayed. The "fog-of-war" in two dimensions is represented as, for example, a color (e.g., black or white) or patterning within the fiber map. graphical representation of the effects of stimulation on the anatomy of a patient population of a particular patient includes representations of a clinical effects map, including variations for indicating differences in therapeutic effect and/or side-effects, which maps can be in two dimensions or three dimensions, and which can include indicia in volumes relative to anatomical tissue and/or an implanted leadwire, and/or relative to or in the form of modeled anatomical fibers. Moreover, information can be categorized, e.g., into therapeutic effects information and side effects information, or other categories (e.g., by type of therapeutic effect and/or type of side effect), with gradations in different graphical indicia being used for the different categories of information, which different indicia are displayed in separate maps or overlaid on each other in a single map.

According to example embodiments of the present invention, a leadwire includes multiple electrodes, for each of which a respective independent current source is provided, by which current can be "steered" longitudinally and/or rotationally about the leadwire for localization of stimulation at points between electrodes (such a point hereinafter referred to as a "virtual electrode"). The electrical variation about a leadwire produced by directional and/or virtual electrodes creates an added layer of complexity concerning stimulation parameters and their effects. Example embodiments of the present invention provide an interface, e.g.,-a visual point-and-click-interface-; that includes a graphical representation of a stimulation parameter for directional and/or virtual electrodes, via which to input settings therefor, and/or via which to obtain and/or output annotations concerning stimulation parameters thereof. According to example embodiments of the present invention, the interface includes controls for gradual directional steering of current about the leadwire, without the requirement for separately setting individual electrical amplitude settings of the individual electrodes, where the steering occurs between actual and virtual electrodes, the interface further providing for the system to receive input of efficacy and adverse side effect information. According to an example embodiment, the obtained input is recorded in association with the settings for which the input was provided, the system thereby generating longitudinal and/or rotational maps of efficacy and side effect information arranged about the leadwire according to the actual and/or virtual electrode positions with which the efficacy and side effect information are associated.

Thus, according to an example embodiment of the present invention, the system performs an iterative process, where each iteration corresponds to a single selected stimulation parameter value, e.g., amplitude, to which value a respective electric field corresponds. For each iteration, the electric field is shifted to various actual and/or virtual electrode locations about the leadwire, and for each of a plurality of the locations to which the respective field of the iteration has been shifted, clinical information regarding therapeutic effect and/or adverse side effect for a stimulation produced by the electric field is recorded. After completion of an iteration, the value of the parameter is changed for a new iteration, in which the shifting and information recording is repeated for the new value. The information obtained during a plurality of the iterations is then usable for construction of a graph on which basis optimal settings are selectable. Such settings include, in an example embodiment, a combination of respective values of the parameter for a selected subset of electrodes of the leadwire.

### BRIEF DESCRIPTION OF THE DRAWINGS

In the drawings, which are not necessarily drawn to scale, like numerals may describe similar components in different views. The drawings illustrate generally, by way of example, but not by way of limitation, various embodiments discussed in the present document.
FIG. 1 shows graphs providing stimulation amplitude information for cylindrically symmetrical electrodes, according to an example embodiment of the present invention.
FIG. 2 shows an example leadwire model that is displayable by a system in a user interface, and further shows relative thereto a coordinate system for indicating amplitude information for directional electrodes, according to an example embodiment of the present invention.
FIG. 3 shows amplitude information graphs for directional electrodes in a three-dimensional perspective, according to an example embodiment of the present invention.
FIG. 4A shows a slider control for rotating a leadwire model, according to an example embodiment of the present invention.
FIG. 4B shows left and right buttons for rotating a leadwire model, according to an example embodiment of the present invention.
FIG. 4C shows a draggable interface component for rotating a leadwire model, according to an example embodiment of the present invention.
FIG. 4D shows a user interface control including a ray that is user-draggable rotationally about a point of origin corresponding to a leadwire and inward and outward with respect to the point of origin, for user modification of electrical parameters, according to an example embodiment of the present invention.
FIG. 5 illustrates user interface components, by user interaction with which a system is configured to receive input of stimulation parameters, according to an example embodiment of the present invention.
FIG. 6 illustrates user interface components, by user interaction with which a system is configured to receive input of annotations regarding stimulation settings, according to an example embodiment of the present invention.
FIG. 7 illustrates user interface components for outputting variations in effect for variations in amplitude in a particular direction, according to an example embodiment of the present invention.
FIG. 8 illustrates a user interface display of information concerning suitable stimulation amplitude parameters for directional electrodes using graphs in a three-dimensional perspective, according to an example embodiment of the present invention.
FIGS. 9A and 9B show user interface displays of the graphs of FIG. 8 in a two-dimensional perspective, according to an example embodiment of the present invention.
FIGS. 10A and 10B show graphs providing stimulation amplitude information for cylindrically symmetrical electrodes, according to an example embodiment of the present invention.
FIG. 11 shows an example leadwire model that is programmed by a system via a user interface, and further shows relative thereto, location and current level information for stimulation at a specified amplitude, according to an example embodiment of the present invention.
FIGS. 12A to 12C show example VOAs all centered at a same location about a leadwire, for which VOAs clinical effects data are recorded, according to an example embodiment of the present invention.
FIG. 12D show a clinical effects graph based on the clinical effects information obtained for the VOAs of FIGS. 12A to 12C, according to an example embodiment of the present invention.
FIGS. 13A to 13C show example VOAs centered at different longitudinal positions of a leadwire, for which VOAs clinical effects data are recorded, according to an example embodiment of the present invention.
FIG. 13D show a clinical effects graph based on the clinical effects information obtained for the VOAs of FIGS. 13A to 13C, according to an example embodiment of the present invention.
FIGS. 14A to 14C show example VOAs about a leadwire for which VOAs clinical effects data are recorded, according to an example embodiment of the present invention.
FIG. 14D show a clinical effects graph based on the clinical effects information obtained for the VOAs of FIGS. 14A to 14C, according to an example embodiment of the present invention.
FIGS. 15A and 15B show example VOAs about a leadwire for which VOAs side effects data are recorded, according to an example embodiment of the present invention.
FIG. 15C shows a graph of side-effect information for stimulation provided at a single longitudinal location along a leadwire with cylindrically symmetrical electrodes overlaid on a graph of therapeutic effect information for stimulation plotted relative to the leadwire, according to an example embodiment of the present invention.
FIG. 16 shows a three-dimensional graph of therapeutic effect information for stimulation provided at a rotational location about a leadwire that includes directional electrodes arranged rotationally about the leadwire, according to an example embodiment of the present invention.
FIG. 17 is a drawing illustrative of a 3-D graph of anatomical regions of a patient including neural elements in a vicinity of a leadwire that includes electrodes for stimulation of the neural elements, which neural elements can be displayed according to therapeutic and/or adverse effect information, according to an example embodiment of the present invention.

### DETAILED DESCRIPTION

FIG. 1 shows an example graphical user interface display of output indicating amplitude information for stimulation using a cylindrically symmetric leadwire. With respect to a cylindrically symmetric leadwire, in an example embodiment, a graph is output in which stimulation amplitude values are plotted, for both a therapy onset curve 101 and side effect onset curve 102, against electrode location, where the electrode locations refer to longitudinal positions of the leadwire. For example, discrete positions along the abscissa can correspond to respective electrodes of the leadwire in their order of arrangement along the leadwire.

Alternatively, different combinations of amplitudes of the electrodes can be set, where each combination can be characterized as having an amplitude setting at a respective longitudinal position of the leadwire, producing a cylindrically symmetric stimulation about the leadwire at that respective longitudinal leadwire position. Positions along the abscissa can represent discrete locations from a first position of the leadwire towards another position of the leadwire, where some of the locations can be those of respective ones of the cylindrically symmetrical electrodes, and others can be other locations corresponding to the combination of stimulation settings of a plurality of the electrodes.

The therapy onset curve 101 indicates amplitude thresholds at which a therapeutic result is expected, depending on the electrode or longitudinal leadwire position at which the respective stimulation amplitude is set. The side effect onset curve 102 indicates a maximum stimulation amplitude at respective electrode or longitudinal leadwire positions, above which the stimulation is expected to cause an adverse side effect. Information on which the curves 101 and 102 are based can include empirically obtained data and/or model-based data. The graphs 101 and 102 can be specific to an indicated desired therapy and/or to an indicated adverse side effect. For example, the graphical user interface, e.g., in a target settings section, can include an input field for inputting a desired therapeutic effect and/or side effect to be avoided, and output a graph such that shown in FIG. 1 as information on which the user, e.g., a clinician, can determine settings to set in the system for producing a stimulation.

Such graphs can be useful for a clinician to eyeball a target range of possible target settings for one or more of the electrodes. For example, the clinician likely would choose to try an amplitude settings that falls at about the center of the shaded area 105 between the curves 101 and 102 since it is that region that is expected to produce a therapeutic effect and to avoid production of an adverse side effect.

However, such a representation does not reflect variations in amplitude at different directions cylindrically about the leadwire using directional electrodes. According to an example embodiment of the present invention, the system and method outputs stimulation amplitude information in a coordinate system in which each plotted data point is identified by a longitudinal position 'z', angle of rotation 'θ', and radius from center 'r', where the longitudinal position is the longitudinal position along the central axis of the leadwire, e.g., a distance from one of the ends, the angle of rotation is an angle between a selected direction extending outward from the leadwire, perpendicularly to the central axis thereof, and the direction in which stimulation is characterized as being produced by an electrode (or combination of electrodes), and radius is a distance from the leadwire along the direction in which the stimulation is characterized as being produced. The radius coordinate corresponds to the stimulation amplitude value, whereas the longitudinal position and angle of rotation information indicates the location of that stimulation. In an example embodiment of the present invention, a computer system provides a graphical user interface in which amplitude settings for a directional electrode leadwire are plotted in curves at planes that are perpendicular to the central axis of the leadwire according to the described coordinate system including longitudinal, angular, and radii values.

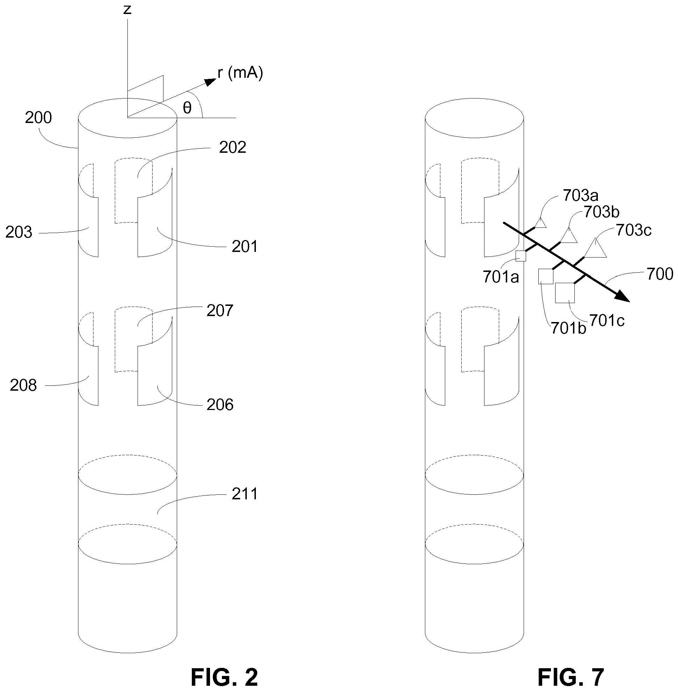

FIG. 2 shows an example leadwire that includes a plurality of directional electrodes. In FIG. 2, the example leadwire 200 includes, at a first longitudinal level of the leadwire, three directional electrodes 201, 202, and 203, at a second longitudinal level of the leadwire, three directional electrodes 206, 207, and 208, and, at a third longitudinal level, a cylindrically symmetrical electrode 211 that is configured for generating a stimulation at approximately equal levels about the leadwire 200. While the directional electrodes are shown to be provided in groups about the leadwire, in an example embodiment, the leadwire 200 includes a circumferential directional electrode that continuously extends around the leadwire, but is controllable for generating stimulations at different levels in different directions from the leadwire.

FIG. 2 further shows a coordinate system in which amplitude values can be plotted using the 'z', 'θ', and 'r' coordinates. The coordinate system is shown relative to the illustrated leadwire 200, thereby showing the meaning of the coordinate values, which represent positional and amplitude information relative to the leadwire 200. Although the leadwire 211 is not a directional leadwire, the same coordinate system can be used for the cylindrically symmetrical leadwire too.

FIG. 3 shows an example user interface display of graphed amplitude settings values using the described coordinate system for a directional leadwire. For example, a graph 302 and/or 304 is drawn with a perspective of being in a two dimensional plane perpendicular to the leadwire 200. Each of the illustrated graphs includes a shape formed by plotted values, for example, representing therapeutic threshold minimum values, i.e., values estimated as the minimum required amplitude for stimulation, where the estimated threshold minimum values vary depending on direction from the leadwire 200 at the longitudinal position of the leadwire 200 at which the plane is drawn. The graphs can alternatively represent maximum amplitude values above which a side effect is estimated to occur. As described below, graphs showing a combination of this information can also be provided.

Stimulation using a combination of electrodes at an one longitudinal level can produce stimulation values characterized by a stimulation at a direction which can be between the electrodes. Similarly, stimulation using a combination of electrodes at a plurality of longitudinal levels can produce stimulation values characterized by a stimulation at a level between electrodes above and below. Therefore, the displayed graphs need not be a longitudinal positions at which there are electrodes (although an alternative example embodiment can be provided in which the graphs are displayed only at longitudinal positions at which at least one electrode is located). In an example embodiment, using graphs plotting stimulations values characterized as occurring between electrodes by combinations of stimulations of those electrodes, the system plots a plurality of two dimensional graphs of stimulation values in a plurality of continuous layers to form a three dimensional graph volume.

In an example embodiment of the present invention, the system displays a model of the leadwire 200, e.g., as shown in FIG. 2 and further displays one or more graphs as shown in FIG. 3. The graphs can be displayed in a separate display area as that in which the model of the leadwire 200 is displayed, or can be displayed overlaid on the model of the leadwire 200. In an example embodiment of the present invention, the system and method of the present invention provides a graphical user interface including view rotation controls, by which a user can rotate the displayed model of the leadwire 200 and the displayed graphs.

For example, FIG. 4A shows a slide bar 400, with the center set at 0°, the right end at +180 °, and the left end at -180 °. A particular longitudinal line at a predetermined point along the circumference of the leadwire is selected as corresponding to 0°. The user can shift a slider control 402 along the slider bar 400, in response to which the system correspondingly rotates the model of the leadwire 200 (and the associated graphs). For example, in an example embodiment, the system shifts the model of the leadwire 200 so that that the selected angular portion of the leadwire 200 is positioned parallel with the surface of the screen in which the user interface is displayed.

Alternatively (or additionally), as shown in FIG. 4B, a right button 405 and a left button 406 can be displayed, which buttons are selectable using an input device, e.g., via point and click, touch, or any other suitably appropriate selection device/method, in response to which selection the model of the leadwire 200 is rotated towards the right or towards the left by a predetermined number of degrees per selection. In an example embodiment, the system displays an indication of the number of degrees the model of the leadwire 200 has been rotated, for example, as shown in FIG. 4B, between the selectable right and left buttons 405/406.

Alternatively (or additionally), as shown in FIG. 4C, the model of the leadwire 200 or a separate leadwire rotation control 410 that is selectable by the user and draggable to the right or to the left is displayed, where, in response to the dragging of the leadwire model 200 or the separate rotation control 410, the system correspondingly rotates the model of the leadwire 200 and the graphs. In an example embodiment, as shown in FIG. 4C, the system displays an indication of the number of degrees the model of the leadwire 200 has been rotated, for example, as shown in FIG. 4C, in a top cross-section of the leadwire representation of the leadwire rotation control 410.

In an example embodiment of the present invention, representations of respective electrodes in the model of the leadwire 200 or in the leadwire rotation control 410 are selectable, in response to which input, the system is configured to obtain user input of one or more settings to be set for the selected electrode. In an example embodiment, the system is configured to display one or more data fields in which to input parameter values for the selected electrode. In an example embodiment, as shown in FIG. 4D, the system is configured to display a ray 415 extending from the selected electrode, which ray 415 the user can select and drag in a direction away from the representation of the leadwire 200 or towards the representation of the leadwire 200, where the system interprets dragging in the direction away from the representation of the leadwire 200 as an input to increase the amplitude setting, and the system interprets dragging back toward the representation of the leadwire 200 as an input to decrease the amplitude. The input can be by a clinician and, in an example embodiment, the system is configured to receive an instruction in response to which the system is configured to apply the modified setting to an implanted pulse generator that causes the leadwire 200 to produce the stimulation. Alternatively or additionally, the user-modification of the settings is for input of stimulation programs for which the system outputs information, e.g., a VOA, and/or other information, based on which the user can select a program to apply to the implanted pulse generator.

In an example embodiment of the present invention, the user interface display including the model of the leadwire 200 further includes a ray, like described ray 415, that extends from the model of the leadwire 200, and the ray is selectable and draggable towards the right and towards the left to modify a directionality of a stimulation, and inwards and outwards with respect to the model of the leadwire 200 to modify an amplitude of the stimulation in the selected direction.

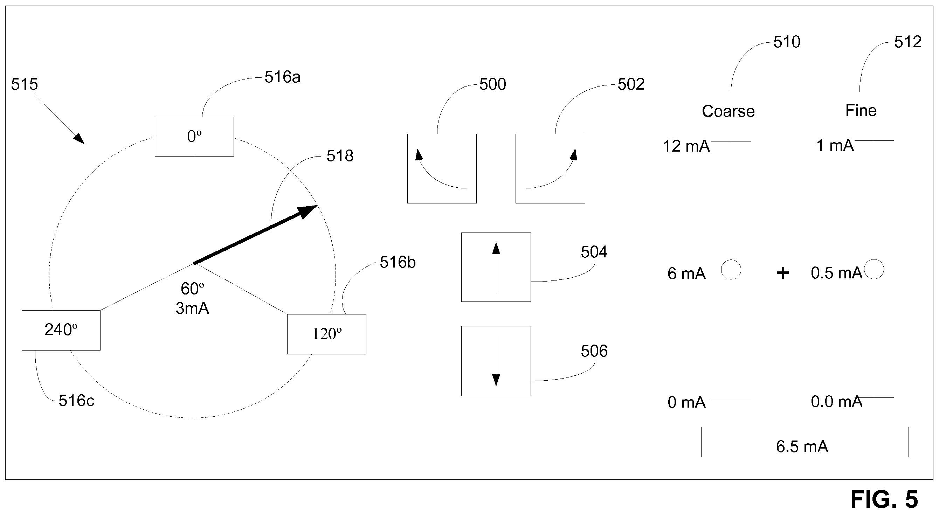

FIG. 5 shows a part of a graphical user interface, according to an example embodiment, that can be displayed in a display device and that includes controls selectable by a user for input of stimulation settings, including a directionality, amplitude, and longitudinal locations relative to the leadwire, for a stimulation. The user interface includes a rotate left button 500, for modifying by the system of the directionality of a stimulation by clockwise rotation about the leadwire by a predetermined incremental amount, responsive to each selection thereof. The user interface further includes a rotate right button 502, for modifying by the system of the directionality of the stimulation by counter-clockwise rotation about the leadwire by a predetermined incremental amount, responsive to each selection thereof. The user interface further includes one or more slider bars by which to modify the amplitude of the stimulation. For example, as shown in FIG. 5, in an example embodiment the user interface includes a coarse slider bar 510 in response to sliding of the slider control of which, the system modifies the amplitude by a first predetermined amount, e.g., a single digit whole number, for each change in position of the slider control; and also includes a fine slider bar 512 in response to sliding of the slider control of which, the system modifies the amplitude by a second predetermined amount smaller than the first predetermined amount, for fine increments between the selected coarse value set by the position of the coarse slider bar 510 and the next higher coarse value corresponding to the position of the coarse slider bar 510 that follows the current position thereof. For example, the coarse slider bar 510 can be set to be shifted between positions 0 and 12, a single whole number at a time, and the fine slider bar 512 can be set to be shifted between positions 0.0 and 0.9, a tenth at a time, so that for whichever value is set by the coarse slider bar 510, the value is further settable to an additional fractional amount.

The user interface further includes an up button 504 and a down button 506, for selection by the user of the longitudinal location along the leadwire at which the stimulation is to occur.

In an example embodiment, as shown in FIG. 5, the user interface further includes a, e.g., two dimensional, settings map 515 that shows present values of the settings with respect to directionality and amplitude of the stimulation. The settings map 515 includes a respective representations 516a-516c of each electrode at a particular longitudinal level. The settings map 515 includes a bar 518 angularly positioned according to the directionality of the stimulations according to the present settings, and whose length corresponds to the presently set amplitude of the stimulation. As the user provides input, e.g., via controls 500, 502, 510, and 512, to modify the directionality and/or amplitude, the bar 518 is rotated and/or shortened or lengthened. In an example embodiment, as shown in FIG. 5, the present angle and amplitude are displayed in the settings map 515.

In an example embodiment of the present invention, the user interface shown in FIG. 5, described above, instead of providing for longitudinally steering the electrical, e.g., current, using the up and down buttons 504 and 506, the system provides for receiving respective input for each longitudinal level of electrodes, each respective input indicating a respective direction and amplitude, e.g., by use of the controls 500, 502, 510, and/or 512, and/or other input controls, e.g., as described herein. For example, for the leadwire 200 as shown in FIG. 2, which includes electrodes 201-203 at a first level, electrodes 206-208 at a second level, and electrode 211 at a third level, the system outputs three user interface sections, e.g., like that shown in FIG. 5, for separately inputting the directional and amplitude settings of the stimulation.

According to a variant of this embodiment, the buttons 504 and 506 are omitted since current steering is not supported. Alternatively, buttons 504 and 506 are provided, but, according to this embodiment, their selections do not cause the above-described current steering, but rather are used for traversing between settings of different electrode levels of the leadwire. For example, the user can use the controls shown in FIG. 5 (or other controls) to set the direction and amplitude for electrodes at a first longitudinal level of the leadwire, and then select one of buttons 504 and 506 to set the settings for the electrodes of, respectively, the next higher or lower longitudinal level of the leadwire.

In an example embodiment of the present invention, the stimulation controls and the settings map 515 are displayed in an interface in which a three-dimensional perspective of a model of the leadwire 200, e.g., as shown in FIG. 2, is also displayed, which model is rotatable, for example, as discussed above with respect to any of FIGS. 4A-4C, and the rotational orientation of the settings map 515 is set by the system to correspond to the rotational orientation of the three-dimensional perspective of the model of the leadwire 200, such that the settings map 515 is rotated when the three-dimensional perspective of the model is rotated.

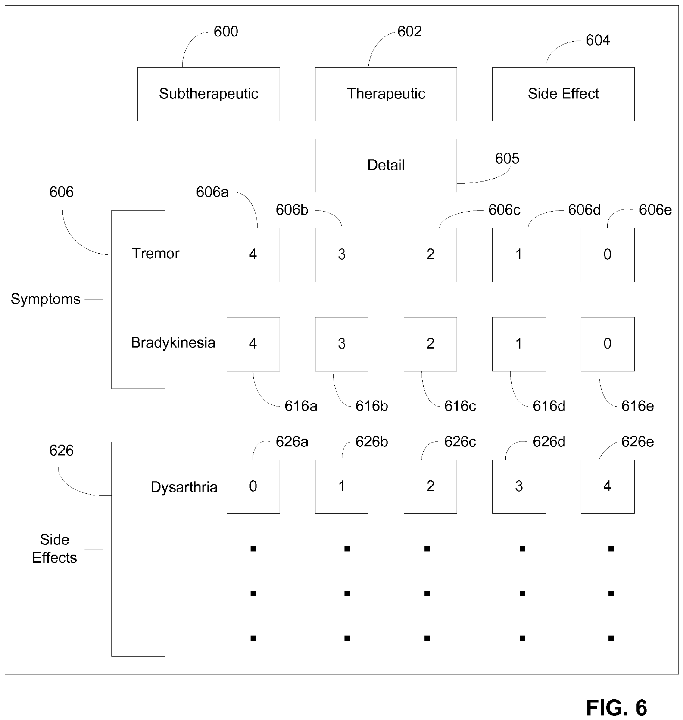

As described above with respect to FIG. 3, according to example embodiments of the present invention, the system displays one or more graphs providing certain threshold (e.g., minimums and/or maximums) information using longitudinal, angular, and radii coordinates, regarding stimulation at various directions about the leadwire. In an example embodiment of the present invention, the system includes an interactive user interface with which a user can interact to input information usable by a processor to generate graphs such as those described with respect to FIG. 3. For example, FIG. 6 shows an example annotation control interface including annotation controls selectable by a user for inputting information concerning presently indicated settings. For example, in an example embodiment, the annotation control interface includes a sub-therapeutic button 600, which, if selected by the user, causes a processor of the system to record in memory information indicating that a stimulation at the presently indicated settings fail to produce a sufficiently therapeutic effect. In an example embodiment, the annotation control interface additionally or alternatively includes a therapeutic button 602, which, if selected by the user, causes a processor of the system to record in memory information indicating that a stimulation at the presently indicated settings fail produce a sufficiently therapeutic effect. In an example embodiment, the annotation control interface additionally or alternatively includes an over limit button 604, which, if selected by the user, causes a processor of the system to record in memory information indicating that a stimulation at the presently indicated settings produces an adverse side effect.

A therapy can cause both a therapeutic effect and an adverse side effect. Therefore, according to an example embodiment of the present invention, the system allows for input indicating both the therapeutic effect and the side effect.

According to an alternative example embodiment of the present invention, the annotation control interface includes a list of symptoms with an associated one or more input fields or selectable controls (e.g., discrete or by slider bar) by which to indicate a degree of therapeutic effect for that respective symptom and/or a list of adverse side effects with an associated one or more input fields or selectable controls (e.g., discrete or by slider bar) by which to indicate a degree to which the respective side effect is caused by the stimulation at the presently indicated settings. For example, as shown in FIG. 6, a symptoms section 606 lists the example symptoms of "tremor" and "bradykinesia" alongside each of which is a respective series of buttons selectable for inputting a respective degree of therapeutic effect for the respective symptom. For example, FIG. 6 shows a set of 5 buttons 606a-606e for inputting respective degrees of therapeutic effect for the symptom of tremor, for example, where selection of button 606a indicates a highest therapeutic effect and selection of button 606e indicates a lowest therapeutic effect (buttons 606b-606d indicating intermediate and progressively decreasing therapeutic effect). FIG. 6 similarly shows a set of 5 buttons 616a-616e similarly operable for the symptom of bradykinesia. FIG. 6 similarly shows a side effect section 626 which lists the example adverse side effect of "dysarthia" alongside which is a respective set of 5 buttons 626a-626e for inputting respective degrees of the adverse side effect of dysarthria, for example, where selection of button 626a indicates a lowest amount of side effect and selection of button 626e indicates a highest amount of side effect (buttons 626b-626d indicating intermediate and progressively increasing side effect).

In an example embodiment of the present invention, the controls for inputting specific therapeutic and side effect information, including identification of particular symptoms for which therapeutic effect is provided and/or identification of particular adverse side effects produced by the therapy, such as controls of sections 606 and 626, and the controls for inputting the more generalized information as to whether a therapeutic effect has been provided and/or a side effect has been produced, such as controls 600-604 are all provided by the system. For example, in an example embodiment of the present invention, the system initially displays controls 600-604, and, responsive to selection of a "details" button or tab 605, the system displays the controls for inputting the information in detailed form. For example, the system updates the interface to simultaneously display all of the controls 600-604 and 606a-626e. Alternatively, the system responsively replaces the generalized controls with the more specific controls. According to either embodiment, the system, in an example embodiment, toggles between the two types of displays responsive to repeated selection of the details button 605.

According to an example embodiment of the present invention, the system is configured to output different graphs as described with respect to FIG. 3 depending on user selectable filter criteria. For example, the user can filter for therapeutic effect related to tremor, in response to which filter the system is configured to output graphs like those shown in FIG. 3 indicating direction-dependent minimum amplitude values for producing a therapeutic effect for tremor, or can similarly filter for therapeutic effect related to bradykinesia. In an example embodiment, without input of a filter criterion, the system outputs a graph based on, for example, minimum amplitude values for producing a therapeutic effect of any kind, i.e., not limited to any one type of selected therapeutic effect.

Similarly, in an example embodiment of the present invention, the user can filter by adverse side effect, e.g., by dysarthia, in response to which filter the system is configured to output graphs like those shown in FIG. 3 indicating direction-dependent maximum amplitude values above which the particular selected side effect is expected to occur. In an example embodiment, without input of a filter criterion, the system outputs a graph based on, for example, maximum amplitude values beyond which any side effect has been recorded to have occurred, i.e., not limited to any one type of selected side effect.

Similarly, instead of or in addition to filtering by type of therapeutic effect and/or side effect, the system provides for filtering based on degree. For example, referring to FIG. 6, the user can filter for only those therapeutic effects indicated by at least a strength of that represented by button 606c, etc. Another example filter criterion is time. For example, the user can filter for graph generation based on input provided in a user-selected time period.

According to an example embodiment, if information is entered indicating the occurrence of a therapeutic effect or side effect, without additional details, e.g., by operation of one or more of the buttons 600-604, without providing additional details concerning degree or type, the system uses such information for the generation of a graph unconstrained by the above-described input criterion of degree and/or type, but does not consider such information for graphs provided in response to a user request constrained by such input criteria.

In an example embodiment of the present invention, the system is configured to output a combination of discrete graphs corresponding to respective types and/or degree. For example, in a plane drawn at a particular longitudinal position of the leadwire, the system outputs one or more graphs corresponding to therapeutic effect for tremor (at one or more degrees of effect) and one or more graphs corresponding to therapeutic effect for bradykinesia (at one or more degrees of effect). The system outputs indicia that identify the effect (and/or degree thereof) to which the different graphs correspond. For example, different colors (and/or hue, saturation, and/or transparency) can be used to represent different effects, and/or different labels can be displayed, e.g., perpetually or when selected or when a pointer is moved over or in close proximity to the graph. The system can similarly generate a plane of overlapping graphs corresponding to different side effects (and/or side effect severities).

In an example embodiment of the present invention, instead of or in addition to a user interface display in which a plurality of graphs for different therapeutic effects, and/or side effects, and/or degrees thereof are included in a single plane, the graphs indicating directional dependency of the amplitude about the leadwire, the system is configured to indicate a variation of stimulation effect (e.g., adverse side effect or therapeutic effect) along a single selected direction from the leadwire as amplitude is increased. For example, FIG. 7 shows a user interface display including a model of a leadwire 200 with a ray 700 including markings at different locations of the ray corresponding to respective distances from the leadwire, which further corresponds to respective amplitude values. The markings can be provided at equal intervals. Alternatively, the markings can be provided wherever there is an appreciable change to the effect (e.g., where the system is programmed to indicate where a predefined difference value or percentage is reached). In an example embodiment, the markings indicate the degree of effect. For example a textual label or other indicia can be used. Further, in an example embodiment, a single ray is marked with different types of indicia for different types of information, e.g., different side effects or therapeutic effects. For example, ray 700 is marked by squares 701a-701c and triangles 703a-703c of different sizes, where squares 701a-701c indicate one type of effect, e.g., therapeutic effect for tremor, and triangles 703a-703c indicate another type of effect, e.g., therapeutic effect for bradykinesia, and where the sizes indicate the degree of such effect, e.g., small shapes indicating a slight effect and large shapes indicating a large effect.

While FIG. 7 shows a single ray 700 in a single direction, in an example embodiment, the system displays a plurality of such rays, each in a respective direction. For example, in an example embodiment, the system generates a display with a plurality of evenly spaced rays cylindrically about the leadwire. In an alternative example embodiment, the system is configured for receiving user input of one or more angles (i.e., directions), and the system accordingly displays respective rays for each of the input angles. In an example embodiment, the system, by default outputs a single ray for each directional electrode.

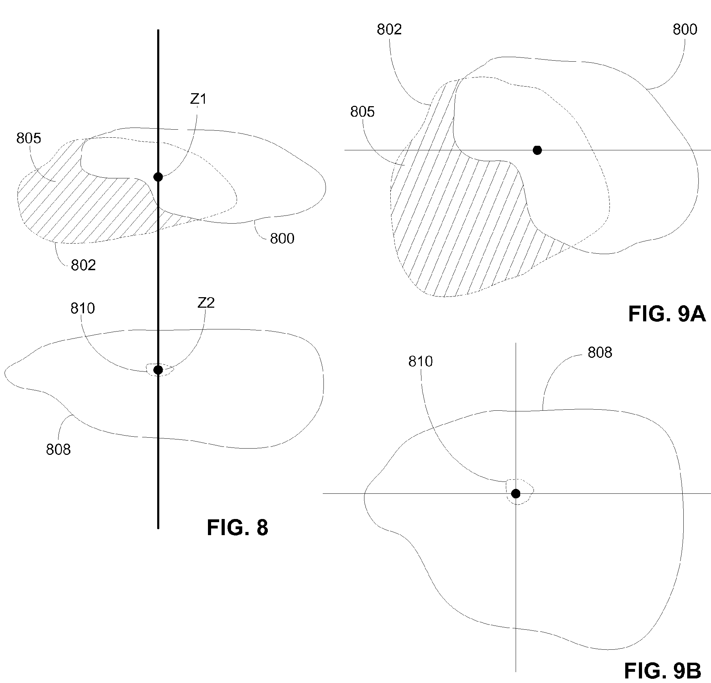

As shown in FIG. 8, in an example embodiment of the present invention, the system is configured to output, in a single plane, graphs for both therapeutic effect and adverse side effect, which graphs can overlap depending on the respective minimum and maximum amplitude values of the graphs in the different directions about the leadwire. A user can thereby determine a range of amplitudes and an angular range about the leadwire at which to set the stimulation. In an example embodiment, the system is configured to mark a graph region determined to be suitable for stimulation based on the relationship between the area of the two graphs (where the graphs do indicate the existence of such a region).

For example, FIG. 8 shows a therapy onset or effect graph 800 and a side effects graph or map 802 within a plane at longitudinal position z1, e.g., along a leadwire. In an example embodiment, the system outputs indicia indicating which graph or map represents therapy onset (or clinical effect) values and which graph or map represents side effects, e.g., advrse side effects, each by line type or color and/or textual indicia, etc. It further includes a cross-hatched region 805, the cross-hatching indicating that region to represent suitable stimulation parameters, e.g., to be suitable for stimulation because it can be stimulated to produce a (threshold minimum) therapeutic effect without producing a (threshold) adverse side effect. Any other suitably appropriate region indicia can be used, e.g., highlighting, coloring, or textual indicia, etc. In the example of FIG. 8, the cross-hatched region is determined to represent suitable parameters, for example, because the parameters corresponding to that region indicated to produce a therapeutic effect without producing an adverse side effect. For example, in the example of FIG. 8, the cross-hatched region is determined to represent suitable parameters because the region lies within the area indicated to be associated with VOAs of the therapeutic effect map 800 and outside of the area indicated to associated with VOAs of the side-effect map 802).

FIG. 8 further shows a therapy onset graph (or therapeutic effects map) 808 and a side effects graph or map 810 within a plane at longitudinal position z2, e.g., along the leadwire. No cross-hatched region is included because there is no suitable range of stimulation parameters at longitudinal position z2, since, in all directions about the leadwire, intolerable side effects set in at lower amplitudes than those at which therapeutic clinical effects are first attained.

It is noted that that there may be certain adverse side effects that are tolerable and there may be certain therapeutic effects that are insignificant. The system is programmed to produce the graphical information for certain predetermined side effects and/or therapeutic effects. Additionally, in an example embodiment, the system includes a user interface via which a user can select one or more side effects and/or one or more therapeutic effects on which basis to generate the graphs or maps.

When the graphs or maps are provided in a three-dimensional perspective about the model of the leadwire 200,, the leadwire model can partially obscure portions of the graphs. Although, as discussed above, example embodiments provide a control for rotating the model, so that the graphs or maps can be rotated and viewed at the different angles, a user may desire to view entire graphs or maps at a time for the respective longitudinal positions at which they are generated. Additionally, when the graphs or maps are provided in a three-dimensional perspective, precise dimensions of the graph or map shape are distorted to account for depth in a two-dimensional display screen, for example, as can be seen by a comparison of the graphs or maps in FIG. 8 and their two-dimensional perspective counterparts shown in FIGS. 9A and 9B. Accordingly, in an example embodiment of the present invention, the system is configured to display the graphs or maps in a two dimensional view, in which the graphs or maps of a single longitudinal position (or other two-dimensional perspective) are displayed such that planes formed by the graphs or maps are parallel to the surface of the display area, e.g., parallel to the surface of a display screen. For example, FIG. 9A shows the graphs or maps 800 and 802 in a two-dimensional view with the leadwire virtually extending perpendicularly to the display screen, and FIG. 9B shows the graphs or maps 808 and 810 in a two-dimensional view with the leadwire virtually extending perpendicularly to the display screen. In an example embodiment of the present invention, a two-dimensional view of graphs or maps is displayed for only a single one of the longitudinal positions of the leadwire at any one time. Alternatively, in an example embodiment, different two-dimensional graph or map views for a plurality of longitudinal positions are simultaneously displayed in different respective display areas of the display screen, e.g., each area including respective indicia indicating the respective longitudinal position to which it corresponds.

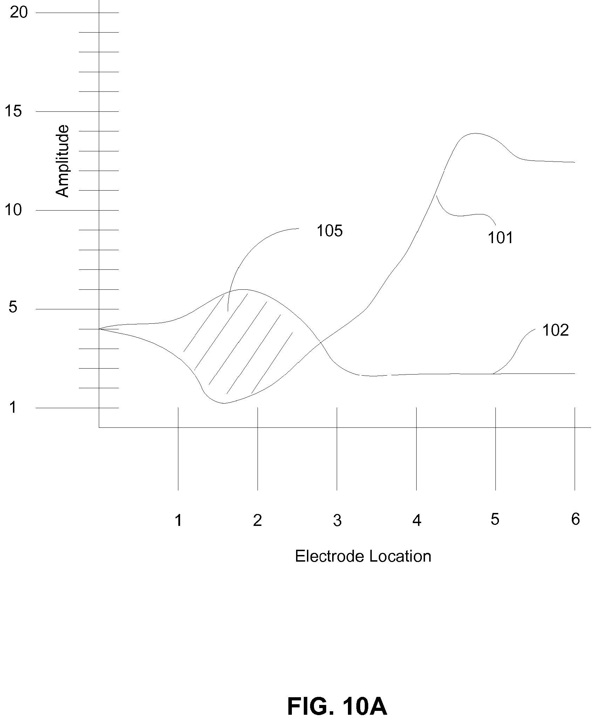

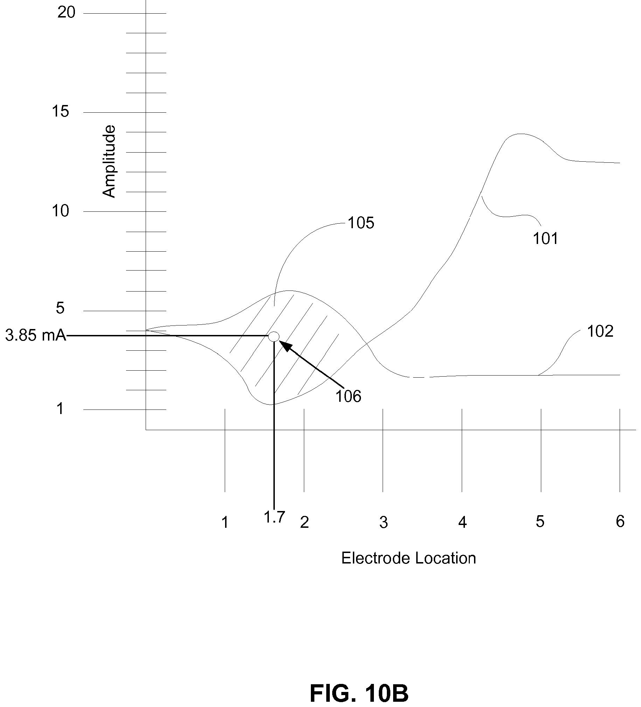

In an example embodiment of the present invention, the system displays a model of the leadwire 200, e.g., as shown in FIG. 2 and further displays one or more graphs or maps as shown in FIGS. 10A and 10B (described below). The graphs or maps can be displayed in a display area separate from that in which the model of the leadwire 200 is displayed, or can be displayed overlaid on the model of the leadwire 200. In an example embodiment of the present invention, the system and method of the present invention provides a graphical user interface including longitudinal steering controls and/or rotational steering controls, by which a user can, for a given fixed parameter (e.g., amplitude, pulse width, frequency, etc.), steer stimulation longitudinally up and down the electrodes of the leadwire and/or rotationally around the electrodes of the leadwire 200, including between actual and/or virtual electrodes, a process hereinafter referred to as e-trolling. At a plurality of arbitrary points (e.g., actual and/or virtual electrode) along the leadwire traversed via e-trolling, the efficacy and side-effects of a stimulation are evaluated. For example, if the patient exhibits undesirable side-effects, the user can annotate the stimulation at the actual or virtual electrode as being in a "side-effect range" by clicking on a button or menu item of the user interface, for example, using controls such as those shown in FIG. 6. Accordingly, if the patient exhibits good symptom relief or therapeutic efficacy, the user can annotate the current setting as being in an "efficacy range" by clicking on a button or menu item.

As explained above, FIG. 5 shows a part of a graphical user interface, according to an example embodiment, that can be displayed in a display device and that includes controls selectable by a user for input of stimulation settings, including a directionality, amplitude, and longitudinal locations relative to the leadwire, for a stimulation. The user interface includes a rotate left button 500, for modifying by the system of the directionality of a stimulation by clockwise rotation about the leadwire by a predetermined incremental amount, responsive to each selection thereof. The user interface further includes a rotate right button 502, for modifying by the system of the directionality of the stimulation by counter-clockwise rotation about the leadwire by a predetermined incremental amount, responsive to each selection thereof. The user interface further includes an up button 504 and a down button 506, for selection by the user of the longitudinal location along the leadwire at which the stimulation is to occur. In the case of a leadwire 200 with only non-directional, circumferential (cylindrically symmetrical) electrodes only an up button 504 and a down button 506, for selection by the user of the longitudinal location for stimulation along the leadwire, are provided or are active. (As noted above, the up and down buttons 504 and 506, according to an alternative example embodiment, provide for selecting a longitudinal level at which to set current information for the electrodes at that level.)

According to an example embodiment, information concerning therapeutic effect and/or adverse side effect is additionally or alternatively obtained using sensors. For example, a sensor can be used to sense patient tremor, speed, stability, heart rate, reaction time, etc., based on which sensed information conclusions concerning therapeutic effect and/or side effect are automatically made and recorded.

Thus, according to an example embodiment of the present invention, a user interface facilitates gradual steering of a current, e.g., at a certain amplitude, frequency, and/or pulse width, about the leadwire, and user annotation of the steered current at various actual and/or virtual electrodes at which the current has been steered, as being in an "efficacy range" or a "side-effects range," by clicking a button or menu item for those electrode locations. According to an example embodiment, the determination of whether the steered current, at an actual and/or virtual electrode at which the current has been steered, is in an "efficacy range" or a "side-effects range" is performed by a processor based on information concerning therapeutic effect and/or adverse side effect additionally or alternatively obtained using sensors. According to an example embodiment, the system records the input (and/or sensor) information in association with the electrode locations to which they correspond, and, based on the recorded information regarding a respective plurality of actual and/or virtual electrodes traversed via e-trolling, generates a curve that connects the annotated values for each such respective identified and annotated actual and/or virtual electrode, thereby graphically identifying the totality of the results (for a given stimulation parameter setting) for a set of electrodes of the leadwire 200 as shown in FIG. 10A. The generated curve includes estimated data for connecting the discrete respective values corresponding to the respective identified and annotated actual and/or virtual electrodes. The set of electrodes can include several cylindrically symmetrical electrodes arranged longitudinally along the leadwire 200 and/or can include several directional electrodes arranged circumferentially around the leadwire 200 at a same longitudinal level on the leadwire 200.

Similar to that shown in FIG. 1, FIG. 10A shows an example graphical user interface display of output indicating amplitude information for stimulation using a leadwire with cylindrically symmetrical circumferential electrodes. The graph is based on the recorded information regarding a respective plurality of actual and/or virtual electrodes traversed via e-trolling and includes a therapy onset (efficacy) curve 101 and a side effect onset (side effect) curve 102 that plot stimulation amplitude values for therapy onset (e.g., meeting a minimum threshold) and side effect onset (e.g., meeting a minimum threshold), against actual and virtual electrode locations, corresponding to longitudinal positions along the leadwire. For example, discrete positions along the abscissa can correspond to respective electrodes and virtual electrodes of the leadwire in their order of arrangement along the leadwire.

According to an alternative example embodiment, a three dimensional graph is used to plot variations in another, e.g., electrical, settings in addition to amplitude. A non-exhaustive list of examples of such parameters include pulse width and frequency. For example, an 'x' axis can correspond to electrode location, a 'y' axis can correspond to amplitude, and a 'z' axis can correspond to the other parameter, so that, for example, different amplitude values are plotted for different values of the other parameter at a same electrode position.

As shown in FIG. 10A, in an example embodiment of the present invention, the system is configured to output, in a single graph, curves for both therapeutic effect (efficacy) and adverse side effect (side effect) measured at multiple actual and/or virtual electrodes of leadwire 200, which curves can intersect depending on the respective minimum and maximum amplitude values of the curves in the different locations about the leadwire. A user can thereby determine a range of amplitudes and locations about the leadwire at which to set the stimulation. In an example embodiment, the system is configured to graphically identify a graph region determined to be suitable for stimulation based on the relationship between the area of the two graphs (where the graphs do indicate the existence of such a region). For example, the processor identifies electrode locations for which associated minimum therapeutic effect amplitude values have been recorded which are lower than side effect amplitude values recorded for the respective electrode locations, identifies the graph area between those values at the respective plotted electrode locations, and displays graphical indicia at the identified graph area. A non-exhaustive list of examples of such indicia include coloring, shading, and/or hatching.

The efficacy curve 101 indicates amplitude thresholds at (or above) which a therapeutic result is expected, depending on the electrode or other longitudinal leadwire position (virtual electrode) at which the respective stimulation amplitude is set. The side effect curve 102 indicates a maximum stimulation amplitude at respective electrode or virtual electrode positions, above which the stimulation is expected to cause an adverse side effect (e.g., above a maximum threshold for such an adverse side effect). Information on which the curves 101 and 102 are based can include empirically obtained data and/or model-based data. The curves 101 and 102 can be specific to an indicated desired therapy and/or to an indicated adverse side effect. For example, the graphical user interface, e.g., in a target settings section, can include an input field for inputting a desired therapeutic effect and/or side effect to be avoided, and output a graph such that shown in FIGS. 10A and 10B as information on which basis the user, e.g., a clinician, can determine settings to set in the system for producing a stimulation. The user can then select the settings for the leadwire electrodes by clicking on the location on the graph that appears to provide the largest therapeutic width, i.e. the location where there is the highest probability of efficacy combined with the lowest probability of an undesired side-effect. According to an example embodiment, the determination of the location where there is the highest probability of efficacy combined with the lowest probability of an undesired side-effect is performed by a processor based on information from curves 101 and 102.

In an example embodiment of the present invention, the graphs are continuously updated as more data points are added via the above-described method of e-trolling. The curve begins as a simple straight line fit between the identified and annotated locations and as more data are added other curve-fitting techniques can be used to better match the recorded values. Curve fitting is the process of constructing a curve, e.g., by use of a mathematical function, which is a best fit to a series of data points, possibly subject to constraints. Curve fitting can involve, e.g., interpolation, where an exact fit to the data is required, or smoothing, in which a "smooth" function is constructed that approximately fits the data. Any suitably appropriate curve fitting function may be used. Accordingly, the output graph, in an example, embodiment, plots information for electrode locations for which therapeutic and/or side effect data has not been obtained, by "filling in" such information based on the information obtained for surrounding electrode locations.

According to an example embodiment, the graphs are also and/or alternatively continuously updated to plot different amplitude values for the therapeutic and or side effect curves for those locations for which input had been previously received, and for which the plotted values had previously reflected such previously obtained input, as more data are added for the previously identified and annotated location. For example, different results may be observed for settings for an electrode location at different times. Because of the variability in measured effects for a subject at a given stimulation location it is beneficial to overwrite any previous side effect threshold values for the location with a lower side effect threshold value for that location so that the user may be more sure about selecting stimulation parameters that will not cause undesired side effects. Likewise, it is beneficial to overwrite any previous efficacy threshold values for the location with a higher efficacy threshold value for that location so that the user may be more sure about selecting stimulation parameters that will produce therapeutic results, e.g. lessen undesired side effects. (Alternatively, averages can be plotted and/or the values to be plotted can be calculated based on a score affected by values of neighboring electrode locations.) Alternatively, time can be used as a third dimension, so that a user is able to see a history of the values.

The two-dimensional graph of FIG. 10A does not reflect variations in amplitude at different directions cylindrically about the leadwire 200 using directional electrodes. Therefore, according to an alternative example embodiment of the present invention, a two-dimensional graph is provided, where the positions along the abscissa represent locations from a first position of the leadwire 200 circumferentially towards another position on the leadwire 200, and where the locations are those of respective ones of actual directional electrodes (e.g., 201-203) and virtual directional electrodes arranged circumferentially on a same longitudinal level of the leadwire 200. In example embodiment, several such graphs are provided, each for a respective longitudinal level along the leadwire 200.

According to an alternative example embodiment, a two-dimensional graph is output, where positions along the abscissa represent longitudinal locations along the leadwire 200, as described above with respect to FIG. 10A, but where one or more of the represented longitudinal locations are respective longitudinal levels at which a plurality of directional electrodes are arranged, with the amplitude information for the therapeutic effect and side effect corresponding to respective combinations of the directional electrodes at the represented longitudinal locations. Others of the represented longitudinal locations can be those at which cylindrically symmetrical electrodes are located. The plotted amplitude levels for the levels at which directional electrodes are located are those generated by one or a combination of the directional electrodes at the respective longitudinal level. Accordingly, some information (i.e., directionality) is missing from the graph for the represented longitudinal levels at which directional electrodes are arranged. For example, in an example embodiment, one or more of the longitudinal positions correspond to those at which cylindrically symmetrical electrodes are located, so that directionality is not a factor, while one or more other longitudinal positions correspond to those at which directional electrodes are located, with directionality not being indicated.

According to an alternative example embodiment of the present invention, the system generates and outputs a three-dimensional graph, with amplitude plotted as radii, as shown in FIG. 3, with multiple layers of such graphs in combination providing a three-dimensional graph volume, as described above, in order to graphically indicate variations of the amplitude values for the therapeutic effect and side effect at different combinations of longitudinal and rotational electrode locations. That is, according to this embodiment, the therapeutic effect and adverse side effect information for the steered locations are plotted to show variations along both longitudinally steered locations and rotationally steered locations.

According to this embodiment, the system and method outputs stimulation amplitude information in a three dimensional coordinate system in which each plotted data point is identified by a longitudinal position 'z', angle of rotation 'θ' and radius from center 'r' as shown by the indicated coordinate system of FIG. 2. The longitudinal position is the longitudinal position along the central axis of the leadwire, e.g., a distance from one of the ends, the angle of rotation is an angle between a selected direction extending outward from the leadwire, perpendicularly to the central axis thereof, and the direction in which stimulation is characterized as being produced by an electrode (or combination of electrodes), and radius is a distance from the leadwire along the direction in which the stimulation is characterized as being produced. The radius coordinate corresponds to the stimulation amplitude value, whereas the longitudinal position and angle of rotation information indicates the location of that stimulation. In an example embodiment of the present invention, a computer system provides a graphical user interface in which amplitude settings for a directional electrode leadwire are plotted in curves at planes that are perpendicular to the central axis of the leadwire according to the described coordinate system including longitudinal, angular, and radii values.

In an example embodiment of the present invention, the system includes a control selectable for toggling between a three dimensional view of the graphs and two dimensional views of the graphs.

As noted above, there may be certain adverse side effects that are tolerable for a certain subject and there may be certain therapeutic effects that are insignificant for said subject. Therefore, in an example embodiment, the system includes a user interface via which a user can select one or more side effects and/or one or more therapeutic effects on which basis to generate the graphs.

Such graphs can be useful for a clinician to eyeball a target range of possible target stimulation settings for one or more of the electrodes. For example, with respect to the graph shown in FIG. 10A, the clinician likely would choose to try stimulation (amplitude + electrode location) settings that fall at about the center of the shaded area 105 between the curves 101 and 102 since it is that region that is expected to produce a therapeutic effect and to avoid production of an adverse side effect.

According to an example embodiment of the present invention, the graph is output as a user-interactive display, where positions within the graph are user-selectable as an instruction to set electrode parameters. For example, as shown in FIG. 10B, in an example embodiment of the present invention, the user interface is configured for the user to point and click on a location within the graph, the selected location being identified in FIG. 10B as location 106, which the user (or system) has determined is likely a location with a high side effect threshold and a large therapeutic width (low efficacy threshold). The system is configured to, responsive to the selection, automatically choose a combination of electrode location and amplitude (and/or other stimulation parameter) associated with the selected point 106 on the graph, for output of the identified parameters in a user interface and/or for setting of the leadwire 200 to produce a stimulation according to such parameters.

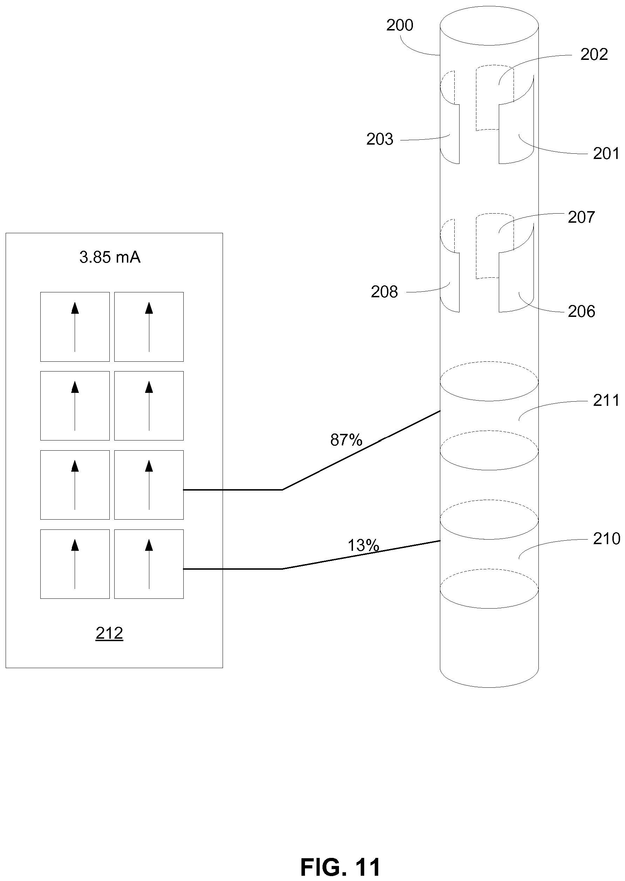

FIG. 11 shows a representation of a plurality of independently controllable current sources 212 for respective ones of the electrodes of the leadwire 200, where the current steering described above, for steering of the current to virtual electrode positions (longitudinally and/or rotationally), is possible by setting the amplitudes of different ones of the current sources 212 to different settings. For example, for settings corresponding to the selected location 106, the system is configured to choose the combination of electrode location and amplitude (and/or other stimulation parameter) associated with the selected point 106 on the graph as a combination of current amplitude values for a respective combination of the current sources 212. The independently controllable current sources 212 for the respective electrodes can be activated in accordance with the stimulation settings associated with selected point 106, e.g., for an amplitude of 3.85 mA and virtual electrode location 1.7, which is closer to the second electrode 211 of leadwire 200 than to the first electrode 210. The stimulation current settings required to localize the stimulation in the area of the virtual electrode associated with the selected point 106 are determined automatically by the system once the point 106 is selected by a user. In an example embodiment of the present invention, the system uses data previously recorded during the e-trolling, regarding the stimulation current settings required to localize the stimulation in the area of the virtual electrode associated with the selected point, if it is available for the selected point. In the example of FIG. 11, electrode 210 receives 13 % of the max current and electrode 211 receives 87% of the max current so that the stimulation is localized at an electrode location corresponding to a virtual electrode located longitudinally between cylindrically symmetrical electrodes 210 and 211 and closer to electrode 211 than to electrode 210.

In an example embodiment of the present invention, a leadwire 200 utilizing a single current source for all of the electrodes of the leadwire 200 is used, and after the user has selected a point associated with stimulation parameters and clinical data on a graph provided according to the user interface described for virtual electrode steering, the system uses pulse interleaving to approximate the stimulation localized in an area of the corresponding virtual electrode. The pulse interleaving uses a single current source that alternates between different current settings at high speed for the different electrodes of the leadwire 200, to provide the different current amplitudes to different ones of the electrodes of leadwire 200 in an alternating manner. In this way, the separate electrodes (e.g., 210 and 211) can receive short-timed pulses from the same current source at different current values (e.g., 13% and 87%) so that stimulation is localized in an area of the corresponding virtual electrode.

As described in detail above, in an example embodiment of the present invention, for a set of stimulation parameters, the system outputs a graphical representation of the parameters, e.g., in the form of a ray extending from a model of the leadwire, where the directionality and length of the ray represents, respectively, a directionality of the stimulation produced by the parameters and the electrical amplitude. In an example embodiment, the system additionally outputs information regarding tissue stimulation produced by the electrical stimulation parameters represented by the array. For example, in an example embodiment, the system displays a first user interface frame identifying one or more of the stimulation parameters and/or including a graphical representation thereof, e.g., in the form of the described ray, and further displays a second user interface section displaying an estimated VOA, e.g., as described in the '330, '312, '340, '343, and '314 applications, corresponding to the indicated and/or represented stimulation parameters.

FIG. 8 shows an example graphical display of output according to an example embodiment of the present invention. According to an example embodiment, the system is configured to output, in a single plane, graphs for both therapeutic effect and adverse side effect reactions to electric field stimulation in a patient's tissue, which graphs can overlap depending on the respective minimum and maximum amplitude values of the graphs in the different directions about the leadwire. The graphs include shaded voxels and a "fog" (e.g., formed using color, pattern, transparency, etc.) is used to indicate voxels for which there is no clinical data yet available. According to example embodiments, a user can use the fog to determine where to "steer" a current field longitudinally and/or rotationally about the leadwire for localization of stimulation in order to obtain new information regarding the effects of stimulation at that region.

In an example embodiment, the system is configured to mark a graph region determined to be suitable for stimulation based on the relationship between the area of the two graphs (where the graphs do indicate the existence of such a region). A user can thereby determine a range of amplitudes and an angular range about the leadwire at which to set the stimulation.

For example, according to an example embodiment, FIG. 8 shows a therapeutic effect map 800 and a side effects map 802 within a plane at longitudinal position z1 along a leadwire. In an example embodiment, the system outputs indicia indicating which map represents clinical effect values and which map represents side effects, each by line type or color and/or textual indicia, etc. It further includes a cross-hatched region 805, the cross-hatching indicating that region to be suitable for stimulation because it can be stimulated to produce a (threshold minimum) therapeutic effect without producing a (threshold) adverse side effect. Any other suitably appropriate region indicia can be used, e.g., highlighting, coloring, or textual indicia, etc. In the example of FIG. 8, the cross-hatched region is determined to represent suitable parameters because the region lies within the area indicated to be associated with VOAs of the therapeutic effect map 800 and outside of the area indicated to associated with VOAs of the side-effect map 802).

FIG. 8 further shows, according to an example embodiment, a therapeutic effects map 808 and a side effects map 810 within a plane at longitudinal position z2 along the leadwire. No cross-hatched region is included because there is no suitable range of stimulation parameters at longitudinal position z2, since, in all directions about the leadwire, intolerable side effects set in at lower amplitudes than those at which therapeutic clinical effects are first attained.

It is noted that that there may be certain adverse side effects that are tolerable and there may be certain therapeutic effects that are insignificant. The system is programmed to produce the graphical information for certain predetermined side effects and/or therapeutic effects. Additionally, in an example embodiment, the system includes a user interface via which a user can select one or more side effects and/or one or more therapeutic effects on which basis to generate the maps.

When the maps are provided in a three-dimensional perspective about the model of the leadwire 200, the leadwire model can partially obscure portions of the graphs. Although, as discussed above, example embodiments provide a control for rotating the model, so that the maps can be rotated and viewed at the different angles, a user may desire to view entire maps at a time for the respective longitudinal positions at which they are generated. Additionally, when the maps are provided in a three-dimensional perspective, precise dimensions of the map shape are distorted to account for depth in a two-dimensional display screen, for example, as can be seen by a comparison of the maps in FIG. 8 and their two-dimensional perspective counterparts shown in FIGS. 9A and 9B. Accordingly, in an example embodiment of the present invention, the system is configured to display the maps in a two dimensional view, in which the maps of a single longitudinal position (or other two-dimensional perspective) are displayed such that planes formed by the maps are parallel to the surface of the display area, e.g., parallel to the surface of a display screen. For example, FIG. 9A shows the maps 800 and 802 in a two-dimensional view with the leadwire virtually extending perpendicularly to the display screen, and FIG. 9B shows the maps 808 and 810 in a two-dimensional view with the leadwire virtually extending perpendicularly to the display screen. In an example embodiment of the present invention, a two-dimensional view of the maps is displayed for only a single one of the longitudinal positions of the leadwire at any one time. Alternatively, in an example embodiment, different two-dimensional map views for a plurality of longitudinal positions are simultaneously displayed in different respective display areas of the display screen, e.g., each area including respective indicia indicating the respective longitudinal position to which it corresponds. Example two dimensional planes, e.g., of a patient brain, which can be displayed include, a plane at a given inferior/superior level and running between the left and right sides and between the anterior and posterior sides, a plane at a given right/left point and running between the superior and inferior sides and between the anterior and posterior sides, and a plane at a given anterior/posterior position and running between the inferior and superior sides and between the left and right sides. Other two-dimensional planes can be similarly selected relative to the implanted leadwire (which may be implanted at an angle relative the anterior/posterior, inferior/superior, and/or left/right directions of the patient anatomy). For example, orthogonal and longitudinal cross-sections of the leadwire can be alternatively selected according to an example embodiment of the present invention.

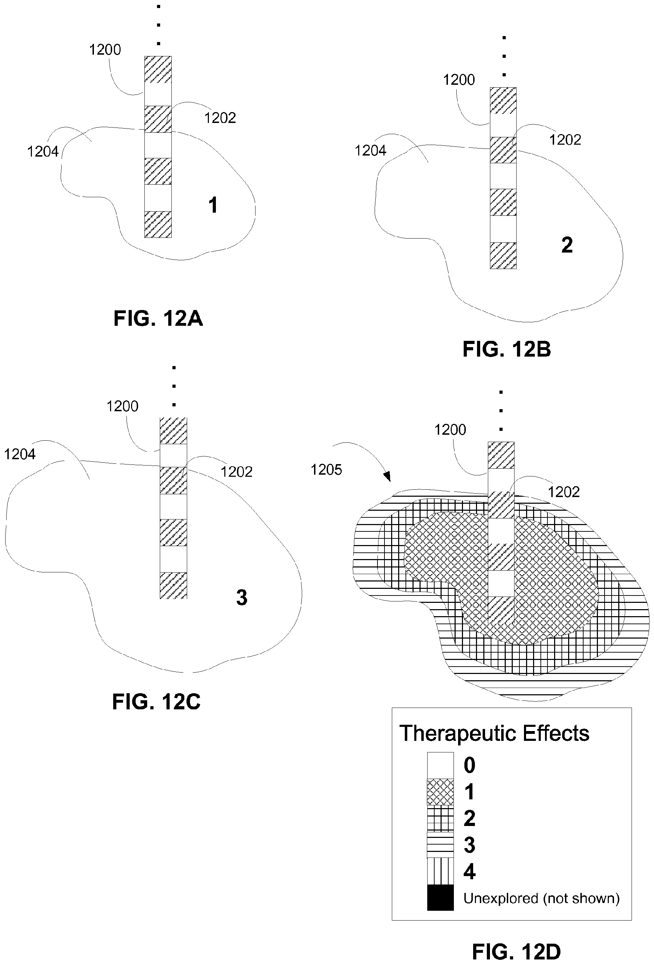

In an example embodiment of the present invention, the system displays a model of the leadwire 1200, and further displays one or more maps as shown in FIGS. 12A-D (as described below). The maps can be displayed in a display area separate from that in which the model of the leadwire 1200 is displayed, or can be displayed overlaid on the model of the leadwire 1200. The maps shown in FIGS. 12A to 12C are VOAs estimated for respective stimulations and for which clinical data has been obtained. At a plurality of points about the leadwire, the therapeutic efficacy and/or adverse side-effects of stimulations are evaluated based on historical stimulations for which those VOAs had been estimated to cover those points. For example, if the patient exhibits undesirable side-effects, the user can annotate a VOA corresponding to the stimulation at which the side-effect(s) occurred as being in a "side-effect range" by clicking on a button or menu item of a user interface. Similarly, if the patient exhibits good symptom relief or therapeutic efficacy, the user can annotate a VOA corresponding to the stimulation at which the therapeutic effect occurred as being in an "efficacy range" by clicking on a button or menu item. It is noted that both a side effect and a therapeutic effect may occur for a same stimulation. Such information can be binary, or can be input as a value from a large scale of values.

According to an example embodiment, information concerning therapeutic effect and/or adverse side effect is additionally or alternatively obtained using sensors. For example, a sensor can be used to sense patient tremor, speed, stability, heart rate, reaction time, etc., based on which sensed information conclusions concerning therapeutic effect and/or side effect are automatically made and recorded.

Similar to that shown in FIGS. 9A-B, FIGS. 12A-C show an example graphical display of a two-dimensional map 1204 of a VOA corresponding to a stimulation using a leadwire 1200 with cylindrically symmetrical electrodes 1202, for which a therapeutic effect had been recorded. While the two-dimensional map 1204 is shown according to a view with the leadwire extending parallel to the display screen, in an example embodiment the cross-sectional plane of a three-dimensional clinical effect map 1204 can be selected by the user for a particular view. For example, the plane can be selected to scrub in the anterior-posterior direction, the medial-lateral direction, and the superior-inferior direction; to scrub in lead-centric views; and to scrub in relative to target anatomical volumes or structures. According to an example embodiment of the present invention, the system displays controls selectable by a user, which controls are respectively associated with predetermined ones of the indicated views. In an example embodiment of the present invention, the two-dimensional planes of the selected view are user-navigable between two dimensional slices. While the VOAs are being described as actually displayed maps, they need not be displayed, but rather the information for such maps are used by the processor to generate the clinical effects map described below with respect to FIG. 12D.

The clinical effects maps are based on the recorded information regarding a respective plurality of actual locations (e.g., voxels that have been part of one or more VOAs corresponding to historically conducted stimulations) for which clinical effect data has already been captured. For example, FIGS. 12A-C show the voxels of VOAs estimated for three different stimulation parameter settings, for which VOAs respective clinical effect values were assigned or determined. Those VOAs can be estimated for different stimulations that have all occurred at different points of time. The VOAs can be of different size or shape. For example, the illustrated VOAs of FIGS. 12A-C are shown to be of different sizes (these are not intended to show actual estimated VOAs, but rather are intended to illustrate that the VOAs can be of different sizes). The VOAs can have overlapping regions and can have non-overlapping regions. For example, certain voxels can be part of all of the VOAs of FIGS. 12A-3D, some in only two of them, and some in only one of them.

For example, the VOAs 1204 of FIGS. 12A-12C are all centered at the same location but are of different sizes in each of FIGS. 12A-C, which could happen if the pulse-width or amplitude is varied. Furthermore, each of the VOAs 1204 represent voxels assigned different clinical effect values indicated by numbers (1-3) within the area of the respective VOA 1204 in each of FIGS. 12A-C. The clinical effect values can be indicated by other indicia, such as coloring, shading, hatching, etc., as described below, and as shown in FIG. 12D.

According to an example embodiment, a combined therapeutic effects map 1205, as shown in FIG. 12D, is generated and displayed based on the information obtained for the respective VOAs 1204 in each of FIGS. 12A-C. In the combined map 1205, clinical effect values are assigned or determined for each voxel based on the collected data available from all available VOAs 1204 including therapeutic effect information for the respective voxel. In the example embodiment shown in FIG. 12D, each voxel is assigned the minimum therapeutic effect value the voxel has been assigned in any of the VOAs 1204 in each of FIGS. 12A-C. (While minimum therapeutic value, discussed herein with respect to FIGS. 12A-15C, refers to the least therapeutic effect, in other terminology, minimum therapeutic value can be thought of as producing the least degree of a symptom for which the therapy is performed, under which alternative terminology, the minimum value is the best therapeutic effect, and according to which terminology, FIG. 12D would be considered to show the maximum values.) Using a metric like Max or Min provides the advantage that only one clinical effect value per voxel need be retained and the values can be readily updated during programming via a straightforward comparison of old and new values for each voxel.

For example, according to an example embodiment in which only maximum values are represented, for each new VOA, for each voxel of the VOA, the system compares the effect value for the current VOA to the effect value stored in association with the voxel, and updates the voxel value only if the new value is greater than the previously stored value. Similarly, according to an example embodiment in which only minimum values are represented, for each new VOA, for each voxel of the VOA, the system compares the effect values for the current VOA to the effect value stored in association with the voxel, and updates the voxel value only if the new value is less than the previously stored value. According to an example embodiment in which the system provides the option for viewing a map based on minimum values and maximum values, the system would store only two values per voxel. However, as discussed below, in other example embodiments, other mathematical functions, e.g., an average, can be used to score a voxel, which may require retaining more, e.g., all, historical values for the voxel.

An advantage of use of the Min metric is that it indicates the volumes one might expect to be required to be stimulated for achieving a certain level of therapeutic effect. Because information pertaining to several VOAs are being displayed together in FIG. 12D, the use of textual information, such as numbers, to indicate the therapeutic effect values, as in FIGS. 12A-C, can be insufficient as there can be insufficient clarity of the volumes to which the text corresponds, and therefore a clinical effects legend is provided, according to an example embodiment, e.g., as shown in FIG. 12D, so that the numerical therapeutic effect values of VOAs 1204 of FIGS. 12A-C can be displayed using other graphical indicia, such as coloring, shading, and/or hatching in the combined therapeutic effect map 1205 of FIG. 12D. A specific graphical indicia (the color black in FIG. 12D) is provided to indicate the "unexplored" areas of the patient anatomy for which no clinical effect information is yet available. These blacked-out areas are not shown in the figures for the purpose of clarity.

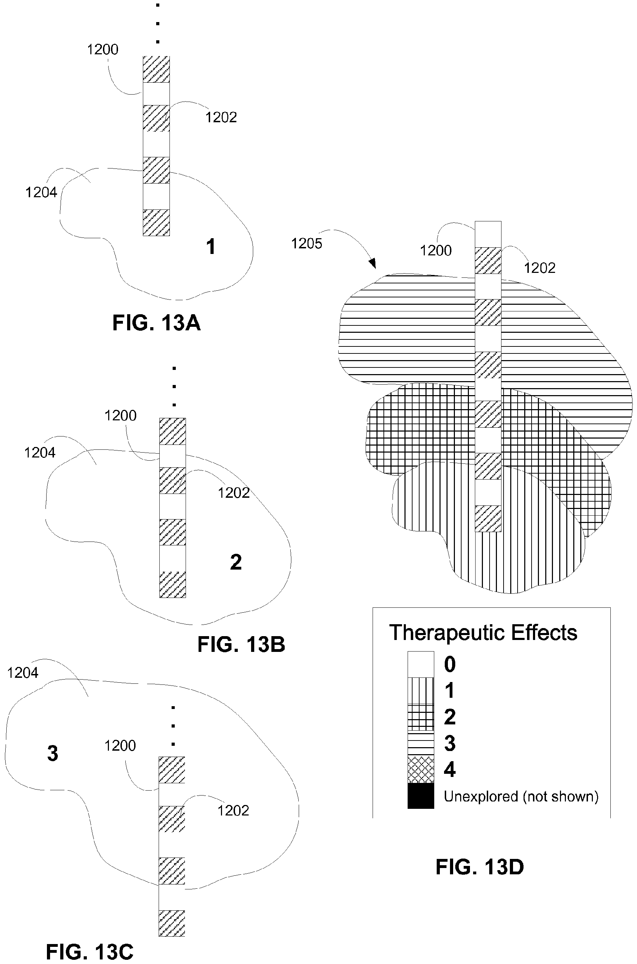

Similar to the VOAs and therapeutic effect map shown in FIGS. 12A-C, FIGS. 13A-C also show example VOAs of different sizes and an example therapeutic effect map. However, unlike FIGS. 12A-C, the VOAs 1204 of FIGS. 13A-C are not centered on the same location in each of FIGS. 13A-C.

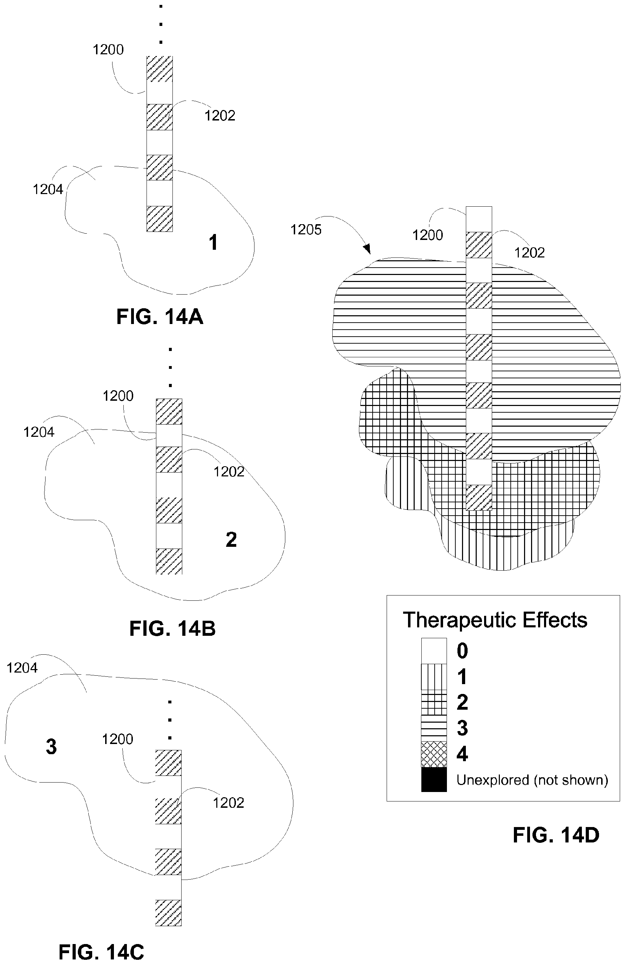

Similar to the VOAs and therapeutic effect map shown in FIGS. 13A-C, FIGS. 14A-C also show example VOAs of different sizes centered at different locations of the leadwire and show an example therapeutic effect map. However, unlike FIG. 13D, the therapeutic effect map 1205 of FIG. 14D is constructed based on maximum historical therapeutic effect values for the voxels, i.e., the maximum therapeutic effect value a respective voxel has been assigned in any of the maps 1204 in each of FIGS. 14A-C. The use of the Max metric for assigning clinical effect values to voxels provides the benefit of better showing where it might be beneficial to center the stimulation on the leadwire 1200. It also indicates the minimum region expected to be required for achieving a certain threshold effect. However, construction of the therapeutic effect map 1205 based on the Max metric can obscure information regarding volumes which would practically be required to be stimulated for obtaining a threshold therapeutic effect. Therefore, according to an example embodiment of the present invention, the system is configured to display maps 1205 based on both the minimum and maximum values, e.g., in different display regions, frames, or windows, or at different times, e.g., depending on a user-selectable option.

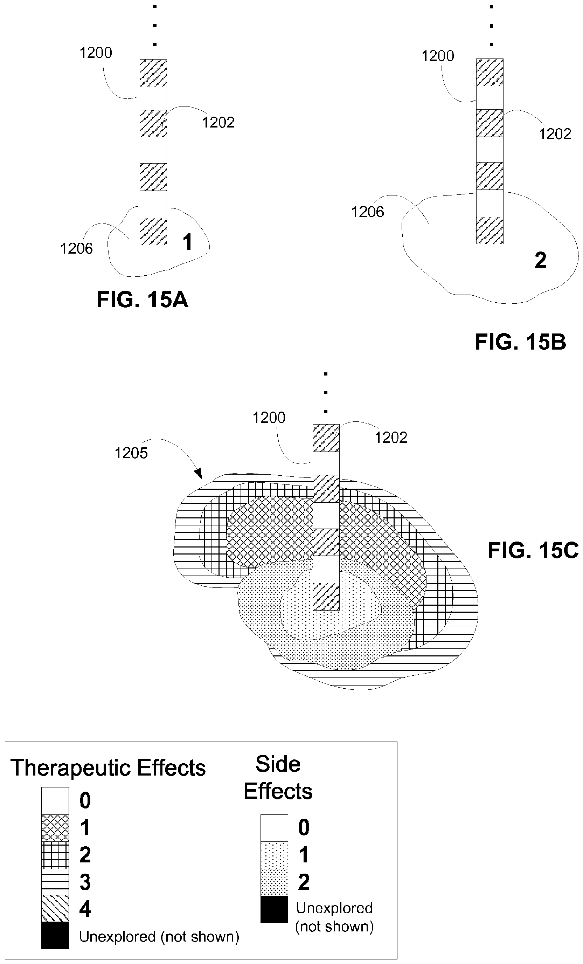

FIGS. 15A-B show an example graphical display of VOAs 1206 estimated for stimulations for which adverse side-effect data has been obtained. (Although, such VOAs are being described separate from the VOAs for which therapeutic effect data are obtained, as noted above, both therapeutic effect and adverse side effect data can be obtained for a same VOA.) For example, FIGS. 15A-B show the estimated VOAs under two different stimulation parameter settings and the respective side-effect values (1 and 2) that were assigned or determined for the estimated VOAs. The VOAs 1206 of FIGS. 15A-B are centered on the same location in each of FIGS. 15A-B and are also of different sizes in each of 15A-B, which as explained above, could happen if the pulse-width or amplitude were varied. The side-effect values can be indicated by other indicia, such as coloring, shading, hatching, etc., as described below. As described with respect to the therapeutic effects maps of FIGS. 12D, 13D, and 14D, an adverse side effects map (not shown alone) can be similarly constructed.

According to an example embodiment, a combined clinical effects map 1205 is formed, as shown in FIG. 15C, based on the information associated with the VOAs 1206 of FIGS. 15A-B and the therapeutic effects map 1205 of FIG. 12D. In the combined clinical map 1205 of FIG. 15C, minimum side-effect values recorded for each voxel based on a plurality of VOAs of which they were a part, and minimum therapeutic effect values recorded for each voxel based on a plurality of VOAs of which they were a part are represented in a combined map. The graphical indicia representing the side effects values are shown to be overlaid on the graphical indicia representing the therapeutic effect values. However, in an example embodiment of the present invention, the side effects indicia are displayed with transparencies so that they do not obscure the graphical indicia representative of the therapeutic effect values. Additionally, while FIG. 15C shows the same types of graphical indicia (hatchings) to be used to show variations in both therapeutic effect and adverse side effect values, in an alternative example embodiment, different types of graphical indicia are used for therapeutic effect and for adverse side effect values. For example, in an example embodiment, variations in color are used for corresponding variations in therapeutic effect scores and variations in hatching are used for corresponding variations in adverse side effect scores. Alternatively, different sets of hatchings can be used for indicating therapeutic effects than those used for indicating adverse side effects. In the example embodiment shown in FIG. 15C, each voxel is assigned the minimum side-effect value the voxel has been assigned in any of the VOAs 1206 of FIGS. 15A-B.

The described figures show independent graphical representations for therapeutic information and side effect information. According to an example embodiment of the present invention, a single score is calculated based on both therapeutic values and side effect values scored for a voxel. For example, an equation can be used by which a therapeutic value positively impacts the voxel's overall score and a side effect value negatively impacts the voxel's overall score. Side effect and therapeutic effect values can be equally weighted or differently weighted. Similarly, while the figures have been described as reflecting minimum or maximum values, in an example embodiment of the present invention, all of a voxel's scores (i.e., the scores of all VOAs of which a voxel has been a part) are considered in an equation which outputs an overall score. In an example embodiment, a combination of these features of integrating both therapeutic and side effect values and of considering all historical voxel values is used.

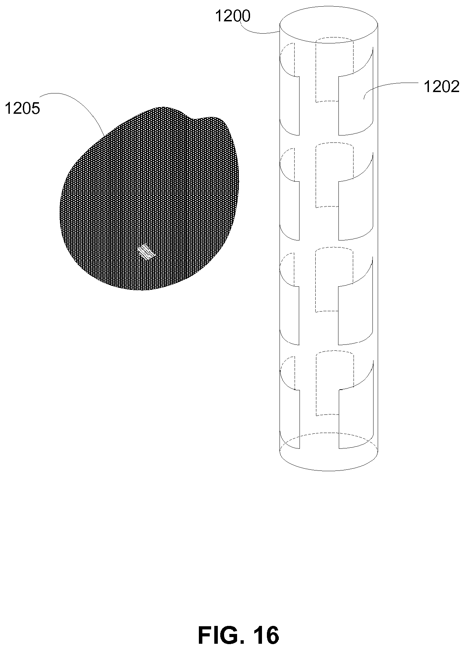

As shown in FIG. 16, in an example embodiment of the present invention, the system is configured to output, a three-dimensional clinical effect map showing a volume of voxels whose scores meet a selected threshold, e.g., a minimum therapeutic effect, a minimum adverse side effect, or a minimum overall score. In FIG. 16 a rotationally asymmetrical leadwire 1200, i.e., including directional electrodes 1202, for which associated clinical effect values have been recorded is shown. The three dimensional clinical effects volume 1205 is shown to be biased towards one side of the leadwire 1200, which can occur particularly using a directional lead, since the stimulations produced by the leadwire 1200 that includes directional electrodes 1202 often are biased towards a particular rotational direction from the leadwire 1200.

The threshold on which basis the volume is generated can be a pre-programmed threshold or can be user-selected. The volume is generated based on a combination of voxels whose scores meet the threshold. In FIG. 16, there is a region separating the displayed volume 1205 and the model of the leadwire 1200. This can occur, for example, because the region between the leadwire and the volume includes voxels associated with many VOAs for which an insufficient score was assigned. For example, there may be many VOAs estimated for stimulations at those intermediate voxels with a low therapeutic effect, but, once a region further away from the leadwire is stimulated, the therapeutic effect for such a stimulation can be much greater. The same can be true with respect to adverse side effects.

Once the voxels whose scores (e.g., minimum or overall score) meet the threshold are determined, the system, according to this example embodiment, displays a representation of the volume, e.g., relative to a model of the leadwire, and/or relative to anatomical structures, e.g., atlas structures or medical image structures.

While threshold volumes are shown in FIG. 16 as a three-dimensional volume, in an alternative example embodiment, the volume can be represented in a two-dimensional plane. Similarly, while the therapeutic and side-effect maps of FIGS. 12D, 13D, 14D, and 15C are shown as two-dimensional planes, in an example embodiment, the system is configured to render also those volumes three-dimensionally.

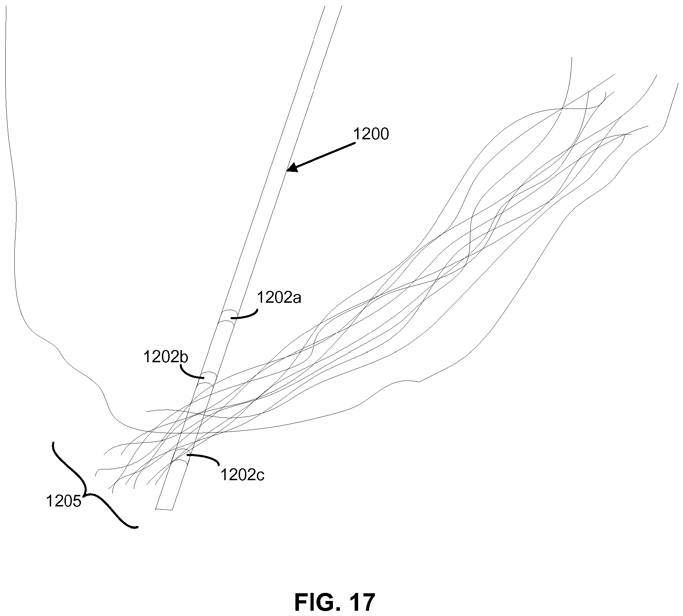

FIG. 17 is a drawing illustrative of an example of a 3-dimensional NEM model of a patient's anatomy, where programmed assumptions about the anatomy of a patient, e.g., the placement of cells, axons and/or terminals of other types of fiber structures with respect to the location of the leadwire, are used for generating the model. For example, FIG. 17 illustrates a representation of an NEM model of a patient's anatomy showing neural populations 1205, of which some can be stimulated using a leadwire 1200 with electrodes 1202a-c. According to an example embodiment, the activated neurons for which clinical effects and/or side-effects data is captured is displayed in a manner that represents those clinical effects and/or side effects data. The clinical effect and/or side-effect values for the activated neurons can be shown via coloring or other graphical indicia as described above with respect to the therapeutic effect, adverse side effect, or combination volume maps. According to this alternative example embodiment, instead providing such graphical indicia on an entire continuous volume, the indicia are placed on individual modeled fibers. For example, different ones of the fibers 1205 are displayed with different colors and/or intensities depending on such data. According to en example embodiment, different parts of a single fiber can be displayed differently depending on the area in which the respective parts of the single fiber lie with respect to the therapeutic and adverse side effect data. According to an alternative example embodiment, each of the fibers is categorized as a whole.

In an example embodiment of the present invention, the described maps are continuously updated as more data points are added so that the "fog" of unexplored areas of an anatomy of a patient (or group of patients) can be removed to provide a clearer and more complete picture of the patient reactions to stimulation. Additionally, the maps can be updated over time to reflect changes in values with which voxels are associated.

In an example embodiment of the present invention, the system includes a control selectable for toggling between a three dimensional view of the graphs and two dimensional views of the graphs.

As noted above, there may be certain adverse side effects that are tolerable for a certain subject and there may be certain therapeutic effects that are insignificant for said subject. Therefore, in an example embodiment, the system includes a user interface via which a user can select one or more side effects and/or one or more therapeutic effects on which basis to generate the graphs.

In an example embodiment of the present invention, the granularity with which the graphical indicia of the historical maps vary is by each individual change in score. According to an alternative example embodiment, a single graphical indicium is used for a range of scores, variations in the graphical indicia occurring between different ranges of scores.

According to an example embodiment of the present invention, the clinical effects data of stimulations performed on a patient are stored in an implanted pulse generator (IPG) used for generating the pulses that cause the leadwire to produce a stimulation and/or are stored on a remote control used for input of the stimulation settings with which the leadwire is programmed. According to an example embodiment, the system access the data from the IPG or the remote control and generates the above-described maps based on the obtained data.

An example embodiment of the present invention is directed to one or more processors, which can be implemented using any conventional processing circuit and device or combination thereof, e.g., a Central Processing Unit (CPU) of a Personal Computer (PC) or other workstation processor, to execute code provided, e.g., on a hardware computer-readable medium including any conventional memory device, to perform any of the methods described herein, alone or in combination, and to generate any of the user interface displays described herein, alone or in combination. The one or more processors can be embodied in a server or user terminal or combination thereof. The user terminal can be embodied, for example, as a desktop, laptop, hand-held device, Personal Digital Assistant (PDA), television set-top Internet appliance, mobile telephone, smart phone, etc., or as a combination of one or more thereof. Specifically, the terminal can be embodied as a clinician programmer terminal, e.g., as referred to in the '330, '312, '340, '343, and '314 applications. Additionally, as noted above, some of the described methods can be performed by a processor on one device or terminal and using a first memory, while other methods can be performed by a processor on another device and using, for example, a different memory.

The memory device can include any conventional permanent and/or temporary memory circuits or combination thereof, a non-exhaustive list of which includes Random Access Memory (RAM), Read Only Memory (ROM), Compact Disks (CD), Digital Versatile Disk (DVD), and magnetic tape.

An example embodiment of the present invention is directed to one or more hardware computer-readable media, e.g., as described above, having stored thereon instructions executable by a processor to perform the methods and/or provide the user interface features described herein.

An example embodiment of the present invention is directed to a method, e.g., of a hardware component or machine, of transmitting instructions executable by a processor to perform the methods and/or provide the user interface features described herein.

The above description is intended to be illustrative, and not restrictive. Those skilled in the art can appreciate from the foregoing description that the present invention can be implemented in a variety of forms, and that the various embodiments can be implemented alone or in combination. Therefore, while the embodiments of the present invention have been described in connection with particular examples thereof, the true scope of the embodiments and/or methods of the present invention should not be so limited since other modifications will become apparent to the skilled practitioner upon a study of the drawings, specification, and the following claims.

## Claims

1. A computer-implemented user interface method for outputting an identification of stimulation settings of a leadwire implanted in, and configured to electrically stimulate tissue of, a patient, the method comprising:
determining, by a computer processor, an angle between a direction of an electrical tissue stimulation according to a first stimulation parameter set and a predefined direction;
converting, by the processor, a value of an electrical amplitude of the stimulation according to the first stimulation parameter set to a radius value; and
displaying, by the processor and in a display device, a single ray (700) whose length
corresponds to the radius value and whose directionality corresponds to the determined angle.

2. The method of claim 1, wherein the leadwire includes a plurality of separately controllable directional electrodes that are arranged circumferentially about a perimeter of the leadwire and that are configured to produce an electrical field in respective directions about the leadwire to cause the electrical tissue stimulation.

3. The method of claim 1, further comprising:
subsequent to the displaying, receiving user input of a second stimulation parameter set; and
responsive to the receipt of the input of the second stimulation parameter set, modifying at least one of the directionality and the length of the single ray.

4. The method of claim 1, further comprising:
outputting information indicating at least one effect of the tissue stimulation caused by the stimulation parameter set represented by the displayed single ray.

5. The method of claim 4, wherein the information and the single ray are simultaneously displayed.

6. The method of claim 5, wherein the information indicates at least one of a type of a clinical effect and a degree of the clinical effect produced by the electrical stimulation.

7. The method of claim 5, wherein the information is output as a graphical estimated volume of activated tissue.

8. A computer system for outputting an identification of stimulation settings of a leadwire implanted in, and configured to electrically stimulate tissue of, a patient, the system comprising:
a display device; and
a computer processor configured to:
determine an angle between a direction of an electrical tissue stimulation according to a first stimulation parameter set and a predefined direction;
convert a value of an electrical amplitude of the stimulation according to the first stimulation parameter set to a radius value; and
display in the display device a single ray (700) whose length corresponds to the radius
value and whose directionality corresponds to the determined angle.

9. The system of claim 8, wherein the leadwire includes a plurality of separately controllable directional electrodes that are arranged circumferentially about a perimeter of the leadwire and that are configured to produce an electrical field in respective directions about the leadwire to cause the electrical tissue stimulation.

10. The system of claim 8, wherein the processor is configured to:
subsequent to the displaying, receive user input of a second stimulation parameter set; and
responsive to the receipt of the input of the second stimulation parameter set, modify at least one of the directionality and the length of the single ray.

11. The system of claim 8, wherein the processor is configured to:
output information indicating at least one effect of the tissue stimulation caused by the stimulation parameter set represented by the displayed single ray.

12. The system of claim 11, wherein the information and the single ray are simultaneously displayed.

13. The system of claim 12, wherein the information indicates at least one of a type of a clinical effect and a degree of the clinical effect produced by the electrical stimulation.

14. The system of claim 12, wherein the information is output as a graphical estimated volume of activated tissue.

15. A non-transitive computer-readable medium on which are stored instructions executable by a processor, the instructions which, when executed by the processor, causes the processor to perform a user interface method for outputting an identification of stimulation settings of a leadwire implanted in, and configured to electrically stimulate tissue of, a patient, the method comprising:
determining an angle between a direction of an electrical tissue stimulation according to a first stimulation parameter set and a predefined direction;
converting a value of an electrical amplitude of the stimulation according to the first stimulation parameter set to a radius value; and
displaying in a display device a single ray (700) whose length corresponds to the radius value
and whose directionality corresponds to the determined angle.

## Patentansprüche

1. Computerimplementiertes Benutzerschnittstellen-Verfahren zur Ausgabe einer Identifikation von Stimulationseinstellungen einer Leitung, die in einen Patienten implantiert ist und dafür konfiguriert ist Gewebe des Patienten elektrisch zu stimulieren, wobei das Verfahren aufweist:
Bestimmen eines Winkels zwischen einer Richtung einer elektrischen Gewebestimulation gemäß einem ersten Stimulationsparametersatz und einer vordefinierten Richtung durch einen Computerprozessor;
Umwandeln eines Werts einer elektrischen Amplitude der Stimulation gemäß dem ersten Stimulationsparametersatz in einen Radiuswert durch den Prozessor; und
Anzeigen eines einzelnen Strahls (700), dessen Länge dem Radiuswert entspricht und dessen Direktionalität dem bestimmten Winkel entspricht, in einer Anzeigevorrichtung durch den Prozessor.

2. Verfahren nach Anspruch 1, wobei die Leitung mehrere getrennt steuerbare Richtungselektroden aufweist, die dem Umfang nach um einen Umfang der Leitung herum angeordnet sind und die konfiguriert sind, um ein elektrisches Feld in jeweiligen Richtungen um die Leitung herum zu erzeugen, um die elektrische Gewebestimulation zu bewirken.

3. Verfahren nach Anspruch 1, das ferner aufweist:
anschließend an das Anzeigen, Empfangen einer Benutzereingabe eines zweiten Stimulationsparametersatzes; und
ansprechend auf den Empfang der Eingabe des zweiten Stimulationsparametersatzes Modifizieren der Direktionalität und/oder der Länge des einzelnen Strahls.

4. Verfahren nach Anspruch 1, das ferner aufweist:
Ausgeben von Information/en, die wenigstens eine Wirkung der Gewebestimulation, die durch den Stimulationsparametersatz bewirkt wird, der durch den angezeigten einzelnen Strahl dargestellt wird, angibt/angeben.

5. Verfahren nach Anspruch 4, wobei die Information/en und der einzelne Strahl gleichzeitig angezeigt werden.

6. Verfahren nach Anspruch 5, wobei die Information/en eine Art einer klinischen Wirkung und/oder ein Maß der klinischen Wirkung, die durch die elektrische Stimulation erzeugt wird, angibt/angeben.

7. Verfahren nach Anspruch 5, wobei die Information/en als ein graphisches geschätztes Volumen von aktiviertem Gewebe ausgegeben wird/werden.

8. Computersystem zur Ausgabe einer Identifikation von Stimulationseinstellungen einer Leitung, die in einen Patienten implantiert ist und dafür konfiguriert ist Gewebe eines Patienten elektrisch zu stimulieren, wobei das System aufweist:
eine Anzeigevorrichtung; und
einen Computerprozessor, der konfiguriert ist, um:
einen Winkel zwischen einer Richtung einer elektrischen Gewebestimulation gemäß einem ersten Stimulationsparametersatz und einer vordefinierten Richtung zu bestimmen;
einen Wert einer elektrischen Amplitude der Stimulation gemäß dem ersten Stimulationsparametersatz in einen Radiuswert umzuwandeln; und
einen einzelnen Strahl (700), dessen Länge dem Radiuswert entspricht und dessen Direktionalität dem bestimmten Winkel entspricht, in der Anzeigevorrichtung anzuzeigen.

9. System nach Anspruch 8, wobei die Leitung mehrere getrennt steuerbare Richtungselektroden aufweist, die dem Umfang nach um einen Umfang der Leitung herum angeordnet sind und die konfiguriert sind, um ein elektrisches Feld in jeweiligen Richtungen um die Leitung herum zu erzeugen, um die elektrische Gewebestimulation zu bewirken.

10. System nach Anspruch 8, wobei der Prozessor konfiguriert ist, um:
anschließend an das Anzeigen eine Benutzereingabe eines zweiten Stimulationsparametersatzes zu empfangen; und
ansprechend auf den Empfang der Eingabe des zweiten Stimulationsparametersatzes die Direktionalität und/oder die Länge des einzelnen Strahls zu modifizieren.

11. System nach Anspruch 8, wobei der Prozessor konfiguriert ist, um:
Information/en auszugeben, die wenigstens eine Wirkung der Gewebestimulation, die durch den Stimulationsparametersatz bewirkt wird, der durch den angezeigten einzelnen Strahl dargestellt wird, angibt/angeben.

12. System nach Anspruch 11, wobei die Information/en und der einzelne Strahl gleichzeitig angezeigt wird/werden.

13. System nach Anspruch 12, wobei die Information/en eine Art einer klinischen Wirkung und/oder ein Maß der klinischen Wirkung, die durch die elektrische Stimulation erzeugt wird, angibt/angeben.

14. System nach Anspruch 12, wobei die Information/en als ein graphisches geschätztes Volumen von aktiviertem Gewebe ausgegeben wird/werden.

15. Nichttransitives computerlesbares Medium, auf dem durch einen Prozessor ausführbare Anweisungen gespeichert sind, wobei die Anweisungen, wenn sie von dem Prozessor ausgeführt werden, bewirken, dass der Prozessor ein Benutzerschnittstellenverfahren zur Ausgabe einer Identifikation von Stimulationseinstellungen einer Leitung, die in einen Patienten implantiert ist und konfiguriert ist, um Gewebe des Patienten elektrisch zu stimulieren, durchführt, wobei das Verfahren aufweist:
Bestimmen eines Winkels zwischen einer Richtung einer elektrischen Gewebestimulation gemäß einem ersten Stimulationsparametersatz und einer vordefinierten Richtung;
Umwandeln eines Werts einer elektrischen Amplitude der Stimulation gemäß dem ersten Stimulationsparametersatz in einen Radiuswert; und
Anzeigen eines einzelnen Strahls (700), dessen Länge dem Radiuswert entspricht und dessen Direktionalität dem bestimmten Winkel entspricht, in einer Anzeigevorrichtung.

## Revendications

1. Procédé d'interface utilisateur mis en œuvre par ordinateur pour fournir en sortie une identification des paramètres de stimulation d'un fil conducteur implanté dans et configuré de manière à stimuler électriquement le tissu d'un patient, le procédé comprenant les étapes ci-dessous consistant à :
déterminer, par le biais d'un processeur informatique, un angle entre une direction d'une stimulation tissulaire électrique selon un premier ensemble de paramètres de stimulation et une direction prédéfinie ;
convertir, par le biais du processeur, une valeur d'une amplitude électrique de la stimulation selon le premier ensemble de paramètres de stimulation, en une valeur de rayon ; et
afficher, par le biais du processeur et dans un dispositif d'affichage, un rayon unique (700) dont la longueur correspond à la valeur de rayon et dont la directionnalité correspond à l'angle déterminé.

2. Procédé selon la revendication 1, dans lequel le fil conducteur inclut une pluralité d'électrodes directionnelles pouvant être commandées individuellement, lesquelles sont agencées circonférentiellement autour d'un périmètre du fil conducteur et lesquelles sont configurées de manière à produire un champ électrique dans des directions respectives autour du fil conducteur en vue d'occasionner la stimulation tissulaire électrique.

3. Procédé selon la revendication 1, comprenant en outre les étapes ci-dessous consistant à :
subséquemment à l'étape d'affichage, recevoir une entrée utilisateur d'un second ensemble de paramètres de stimulation ; et
en réponse à la réception de l'entrée du second ensemble de paramètres de stimulation, modifier au moins une caractéristique parmi la directionnalité et la longueur du rayon unique.

4. Procédé selon la revendication 1, comprenant en outre l'étape ci-dessous consistant à :
fournir en sortie des informations indiquant au moins un effet de la stimulation tissulaire occasionnée par l'ensemble de paramètres de stimulation représenté par le rayon unique affiché.

5. Procédé selon la revendication 4, dans lequel les informations et le rayon unique sont affichés simultanément.

6. Procédé selon la revendication 5, dans lequel les informations indiquent au moins l'un des éléments parmi un type d'un effet clinique et un degré de l'effet clinique produit par la stimulation électrique.

7. Procédé selon la revendication 5, dans lequel les informations sont fournies en sortie sous la forme d'un volume estimé graphique de tissu activé.

8. Système informatique destiné à fournir en sortie une identification de paramètres de stimulation d'un fil conducteur implanté dans et configuré de manière à stimuler électriquement le tissu d'un patient, le système comprenant :
un dispositif d'affichage ; et
un processeur informatique configuré de manière à :
déterminer un angle entre une direction d'une stimulation tissulaire électrique selon un premier ensemble de paramètres de stimulation et une direction prédéfinie ;
convertir une valeur d'une amplitude électrique de la stimulation selon le premier ensemble de paramètres de stimulation, en une valeur de rayon ; et
afficher, dans le dispositif d'affichage, un rayon unique (700) dont la longueur correspond à la valeur de rayon et dont la directionnalité correspond à l'angle déterminé.

9. Système selon la revendication 8, dans lequel le fil conducteur inclut une pluralité d'électrodes directionnelles pouvant être commandées individuellement, lesquelles sont agencées circonférentiellement autour d'un périmètre du fil conducteur et lesquelles sont configurées de manière à produire un champ électrique dans des directions respectives autour du fil conducteur en vue d'occasionner la stimulation tissulaire électrique.

10. Système selon la revendication 8, dans lequel le processeur est configuré de manière à :
subséquemment à l'étape d'affichage, recevoir une entrée utilisateur d'un second ensemble de paramètres de stimulation ; et
en réponse à la réception de l'entrée du second ensemble de paramètres de stimulation, modifier au moins une caractéristique parmi la directionnalité et la longueur du rayon unique.

11. Système selon la revendication 8, dans lequel le processeur est configuré de manière à :
fournir en sortie des informations indiquant au moins un effet de la stimulation tissulaire occasionnée par l'ensemble de paramètres de stimulation représenté par le rayon unique affiché.

12. Système selon la revendication 11, dans lequel les informations et le rayon unique sont affichés simultanément.

13. Système selon la revendication 12, dans lequel les informations indiquent au moins l'un des éléments parmi un type d'un effet clinique et un degré de l'effet clinique produit par la stimulation électrique.

14. Système selon la revendication 12, dans lequel les informations sont fournies en sortie sous la forme d'un volume estimé graphique de tissu activé.

15. Support non transitoire lisible par ordinateur sur lequel sont stockées des instructions exécutables par un processeur, dans lequel les instructions, lorsqu'elles sont exécutées par le processeur, amènent le processeur à mettre en œuvre un procédé d'interface utilisateur pour fournir en sortie une identification de paramètres de stimulation d'un fil conducteur implanté dans et configuré de manière à stimuler électriquement le tissu d'un patient, le procédé comprenant les étapes ci-dessous consistant à :
déterminer un angle entre une direction d'une stimulation tissulaire électrique selon un premier ensemble de paramètres de stimulation et une direction prédéfinie ;
convertir une valeur d'une amplitude électrique de la stimulation selon le premier ensemble de paramètres de stimulation, en une valeur de rayon ; et
afficher, dans un dispositif d'affichage, un rayon unique (700) dont la longueur correspond à la valeur de rayon et dont la directionnalité correspond à l'angle déterminé.
